Europäisches Patentamt

European Patent Office

Office européen des brevets

(11) Publication number: **0 514 927 A1**

## EUROPEAN PATENT APPLICATION

(21) Application number: **92108687.2**

(22) Date of filing: **22.05.92**

(51) Int. Cl.5: **C12Q 1/68**, //G01N35/00

(30) Priority: **24.05.91 US 705510**

(43) Date of publication of application:
**25.11.92 Bulletin 92/48**

(84) Designated Contracting States:
**AT BE CH DE DK ES FR GB GR IT LI LU MC NL PT SE**

(71) Applicant: **Gilbert, Walter**
**15 Gray Gardens West**
**Cambridge, Massachusetts 02138(US)**

(72) Inventor: **Gilbert, Walter**
**15 Gray Gardens West**
**Cambridge, Massachusetts 02138(US)**

(74) Representative: **Vossius & Partner**
**Siebertstrasse 4 P.O. Box 86 07 67**
**W-8000 München 86(DE)**

(54) Method and apparatus for rapid nucleic acid sequencing.

(57) An automated nucleic acid sequencer is provided comprising an oligomer synthesizer, a membrane unit array, a detector and a central computer. The synthesizer synthesizes and labels multiple oligomers of [arbitrary] predicted sequence, which are transported to selected membranes contained within a membrane unit array, where they are hybridized to sequencing patterns bound to the membranes. A detector detects the hybridized sequencing patterns and sends descriptions of those patterns to the central computer, which analyzes those descriptions to construct a nucleic acid sequence, predicts a next set of oligomers for subsequent hybridizations, and selects corresponding membranes for hybridization with each predicted oligomer. Under computer control, synthesis of multiple oligomers, hybridization within multiple membranes, detection of the resulting patterns on multiple membranes, prediction of next oligomers, and selection of corresponding membranes, proceed simultaneously in accordance with the steps of a method of automated sequencing. Also provided is a method for automated nucleic acid sequencing.

This invention relates to a method and apparatus for automating the steps of nucleic acid sequencing. More particularly, this invention relates to a computer controlled sequencer that performs an iterative process of synthesizing sets of predicted oligomers, hybridizing each synthesized oligomer to sequencing patterns immobilized on a selected membrane contained in an array of membrane units, detecting the hybridized patterns, analyzing those patterns to construct nucleic acid sequences, and predicting next sets of oligomers and selecting corresponding membranes for subsequent iterations of synthesis, hybridization, detection, analysis, prediction and selection.

For more than a decade, scientists have utilized nucleic acid sequencing techniques to identify and study genes and their products. Traditional DNA sequencing techniques utilizing chemical cleavage (Maxam and Gilbert, Proc. Natl. Acad. Sci. USA, 74, pp. 560-564 (1977)) and chain termination (Sanger et al., Proc. Natl. Acad. Sci. USA, 74, pp. 5463-5467 (1977)) are well known to those skilled in the art. Although different in principle, both techniques generate separate populations of labelled oligonucleotides that begin from a fixed point and terminate randomly at a particular type of residue. Gel electrophoresis of such populations generates "ladders" from which the DNA sequence can be read.

The academic and commercial demand for nucleic acid sequence information has increased to the point that it is now desirable to begin sequencing not just isolated genes, but entire genomes of microorganisms, plants and animals. For instance, sequence information from the human genome, provided by the Human Genome Project, is expected to enable vast progress in disease diagnosis, treatment and drug design. A variety of sequencing methods are currently being employed in genomic sequencing efforts as well as in small-scale sequence determinations.

"Genomic sequencing," which was first developed in 1984, involves direct chemical modification of unlabelled genomic DNA, combined with complete restriction enzyme digestion and size separation on a denaturing gel. Numerous co-migrating sets of DNA sequence ladders are produced that, after transfer to a membrane, can be visualized independently by probing the membrane with a single-stranded labelled probe specific for one end of one restriction fragment within the genome [G.M. Church and W. Gilbert, "Genomic Sequencing", Proc. Natl. Acad. Sci. USA, 81, pp. 1991-95 (1984)]. Because the DNA sequence is determined directly from genomic DNA, without the necessity of intervening cloning steps, this technique also permits study of naturally occurring DNA modifications and DNA-protein interactions.

Recently, two new techniques have been developed that allow faster and more efficient DNA sequencing. The first, "multiplex DNA sequencing," involves formation of artificial genomes from mixtures of sonicated genomic fragments inserted into multiple vectors adjacent to various "tag" oligonucleotides. The DNA is chemically cleaved, electrophoresed, transferred to membranes, and then probed repeatedly with oligonucleotides complementary to the "tag" sequences in the vectors [G.M. Church and S. Kieffer-Higgins, "Multiplex DNA Sequencing," Science, 240, pp. 185-88 (1988)].

The second technique, "multiplex walking," is described in O. Ohara et al., "Direct Genomic Sequencing Of Bacterial DNA: The Pyruvate Kinase I Gene Of Escherichia Coli," Proc. Natl. Acad. Sci. USA, 86, pp. 6883-87 (1989). This technique utilizes the genomic sequencing technique described above. When a labelled oligomer is hybridized to a membrane containing the chemical cleavage products of restriction digested DNA, sequence appears wherever the oligonucleotide lies near a restriction site. For each restriction digest, sequence may be readable in either the 5' or the 3' direction, depending on the location of the oligonucleotide relative to the restriction site. Confirming sequence appears in the lanes derived from other restriction digests. Thus, each probing reveals the sequence of one strand of DNA extending in one or both directions from the location of the oligonucleotide. Sequence can be read from the hybridization site of the probe along the length of the restriction fragment, until extraneous sequences that originate from the next restriction site obscure the sequence ladder. Sequence from the complementary strand is generated on separate membranes by using complementary oligonucleotides.

After the sequence from the first hybridization is determined, new probes are synthesized, with their sequences being derived from that portion of DNA that contains restriction sites and that is located near the 5' and 3' ends of the newly generated sequence. When the membranes are rehybridized with the new probes, additional sequence ladders are revealed. Repeated cycles of oligomer probe synthesis and subsequent reprobing permit sequence walking along the genome. [See O. Ohara et al., supra.]

Despite the development of the DNA sequencing techniques described above, the cost and time required to sequence the number of nucleic acids now being studied presents a great hindrance to current research efforts. There exists, therefore, a great demand for faster, more efficient, and less expensive ways to sequence DNA.

The present invention solves the problems referred to above by providing a method and apparatus for automating nucleic acid sequencing and for increasing the speed and efficiency of nucleic acid sequencing.

It is an object of this invention to provide an automated sequencer that will perform nucleic acid sequencing through an iterative process of synthesizing multiple predicted oligomers and hybridizing at least two of those oligomers to sequencing patterns bound to respective selected membranes.

It is a further object of this invention to provide an automated sequencer comprising an oligomer synthesizer capable of synthesizing multiple oligomers simultaneously.

It is a further object of this invention to provide an automated sequencer comprising a plurality of membrane units, each of which includes a membrane containing immobilized nucleic acid sequencing patterns, the membrane being bonded to a rigid backing plate to enable multiple iterations of hybridization, washing and stripping of the membrane, and to enable automated movement of the membrane.

It is a further object of this invention to provide an automated sequencer comprising a membrane unit array that stores a plurality of membrane units that are individually selected for automated hybridization with a predicted oligomer, each stored membrane unit being separately connected to an automated fluid transportation system for providing oligomers and fluids to hybridize, wash and strip the membrane contained therein.

It is a further object of this invention to provide an automated sequencer comprising a detector to detect the hybridized sequencing patterns on the membranes and to provide descriptions of such patterns to a computer.

It is a further object of this invention to provide an automated sequencer comprising a computer that controls and coordinates the iterative, multiple operations of: synthesis of predicted oligomers; hybridization of each oligomer to sequencing patterns immobilized on selected membranes; detection of hybridized patterns on the membranes; interpretation of detected patterns to construct nucleic acid sequences; prediction and synthesis of next oligomers; and selection of corresponding membranes for subsequent iterations of hybridization, detection, interpretation, prediction, synthesis, and selection.

It is a further object of this invention to provide an automated sequencer that will perform nucleic acid sequencing through an iterative process of synthesizing multiple predicted oligomers, hybridizing at least two of those oligomers to sequencing patterns immobilized on selected membranes contained within a plurality of membrane units, detecting the hybridized sequencing patterns, interpreting the patterns to construct nucleic acid sequences, and predicting next oligomers for subsequent iterations of the process.

It is a further object of this invention to provide a method for automated sequencing of a nucleic acid using a multiplex walking technique comprising the steps of synthesizing oligomers of predicted sequence, hybridizing synthesized oligomers to sequencing patterns bound to selected membranes, detecting hybridized sequencing patterns, analyzing detected patterns to construct a nucleic acid sequence, predicting the sequence of and synthesizing next oligomers and selecting membranes for subsequent hybridization, detection and analysis.

In accordance with the invention, an automated sequencer and method for automated sequencing are provided. The automated sequencer comprises an oligomer synthesizer, a membrane unit array, a detector and a central computer.

The oligomer synthesizer simultaneously synthesizes multiple oligomers of any chosen sequence in accordance with instructions received from the central computer. The synthesized oligomers are then purified and labelled by the automated synthesizer.

The membrane unit array contains a plurality of reusable membrane units. The membrane units, which are constructed separately, and preferably in advance of oligomer synthesis, include a membrane containing immobilized nucleic acid sequencing patterns, a backing plate to which the membrane is bound, a sheet that is attached to the backing plate and covers the membrane to form an enclosure, and a series of inlet and outlet tubes for permitting fluids to be transported into and out of the enclosed space.

A fluid transportation system conducts each labelled oligomer to a selected membrane unit contained within the membrane unit array for hybridization. The fluid transportation system also conducts a series of fluids into and out of the membrane units for hybridizing, washing and stripping the membranes contained therein. An opposing moveable plate is also provided to compress the cover sheet to restrict the layer of fluid between the membrane and the cover sheet to a desired thickness. These steps can proceed independently and simultaneously in the various membrane units contained in the membrane unit array.

The detector detects the sequencing patterns on the membranes elucidated by hybridization of the labelled oligomers, and provides descriptions of those patterns to the central computer. A detection element may be inserted between the cover sheet and the moveable plate to enable detection of the hybridized sequencing patterns, or the membrane unit may be extended out of the membrane unit array to be presented to a detector for detection of the hybridized patterns on the membrane. After detection, each detected membrane is stripped of the hybridized oligomer to enable subsequent hybridization with a next oligomer.

The central computer analyzes the detected patterns to construct a nucleic acid sequence, predicts a next set of oligomers for extending and checking the determined nucleic acid sequence, directs the synthesizer to synthesize the next set of oligomers, and determines which membrane each predicted oligomer will be directed to for hybridization. The central computer further serves to control the operation of the components of the automated sequencer in a coordinated manner, reducing the time required to determine the nucleic acid sequence. Under computer control, synthesis of multiple oligomers, transportation of oligomers and fluids, hybridization within multiple membrane units, and detection of the resulting patterns on multiple membranes and construction of nucleic acid sequence data proceed rapidly and efficiently in accordance with the steps of the method of automated sequencing described herein.

The above and other objects and advantages of this invention will be apparent from consideration of the following detailed description, taken in conjunction with the accompanying drawings:

FIG. 1 is a schematic representation of the principle components of the sequencer;

FIG. 2 is a combined block-pictorial diagram of one embodiment of the synthesizer of the present invention;

FIG. 3 is a cross-sectional view of a portion of the synthesizer;

FIG. 4a is a top elevational view of the trough module;

FIG. 4b is a front elevational view of the trough module;

FIG. 4c is a rear elevational view of the trough module;

FIG. 4d is an end elevational view of the trough module;

FIG. 5 is an end view of a portion of the synthesizer;

FIG. 6 is a bottom perspective view of the trough module and associated structures;

FIG. 7 is a diagram of the reagent reservoir and drive system;

FIG. 8 is a partially exploded side elevational view of a solenoid-piston-reagent-tip assembly;

FIG. 9 is a wiring diagram of the OM-915 controller terminal board;

FIG. 10 is a schematic diagram of the valve block;

FIG. 11 is a schematic diagram of the synthesizer enclosure wiring;

FIG. 12 is a flow chart for a computer program suitable for use with the synthesizer;

FIG. 13 is a simplified perspective diagram showing the placement of the solenoid array with respect to the trough module;

FIG. 14 is a perspective view of a membrane unit array with one membrane unit extending therefrom for presentation to a second embodiment of the detector;

FIG. 15 is a second perspective view of a membrane unit array with one membrane unit extending therefrom and engaged with a second embodiment of the detector;

FIG. 16 is a perspective view of the preferred embodiment of the membrane unit and opposing cover plate;

FIGS. 17a and 17b are sectional views of the membrane unit and opposing cover plate showing their released and compressed positions during a cycle of hybridizing, washing and stripping;

FIG. 18 is a schematic representation of one embodiment of the fluid transportation system;

FIGS. 19a and 19b are sectional views of the membrane unit and opposing cover plate showing their released and compressed positions in connection with a first embodiment of the detector.

This invention provides an automated nucleic acid sequencer that is able automatically to perform the steps of multiplex genomic walking, thus greatly enhancing the efficiency and speed of nucleic acid sequencing. According to this invention, nucleic acid sequences are determined in an automated, iterative process of synthesis of multiple oligomers, hybridisation of said oligomers to sequencing patterns immobilized on multiple membranes contained within a membrane unit array, detection and analysis of hybridized sequencing patterns, construction of a portion of a nucleic acid sequence, and analysis of the newly constructed sequence to predict a next set of oligomers to be synthesized, hybridized, etc., in order to extend and check the sequence.

Information obtained from the nucleic acid sequence data generated by the apparatus and method of this invention is useful to prepare nucleic acid molecules, including DNA, cDNA and RNA molecules, which have at least a portion of their sequence based on the aforementioned nucleic acid sequence data. Such prepared nucleic acid molecules may be useful in a variety of other methods, for example: methods comprising the steps of cloning, isolation, or manipulation of particular genetic sequences in tissues or other analytes; methods of determining the presence or absence of particular genetic sequences; methods of determining the presence or absence of any molecule or other thing associated with the presence or absence of particular genetic sequences; methods of diagnosis and detection of pathological or nonpathological conditions which may be associated with the presence or absence of particular genetic sequences or the presence or absence of things associated with those sequences; methods comprising the step of introducing a particular genetic sequence into a host cell or organism; and any other method which comprises a step requiring knowledge and possession of a particular genetic sequence.

As used herein, a "nucleic acid sequencing pattern" is any pattern of nucleic acid molecules bound to a membrane and capable of being hybridized with a nucleic acid probe to reveal a nucleic acid sequence ladder.

As used herein, a "nucleic acid sequence ladder" is the pattern of nucleic acid molecules which could be identified by an effective end label and which results from size separation of the products of a sequencing reaction, so that the order of elements in the pattern corresponds to the positions of the bases in a nucleic acid sequence.

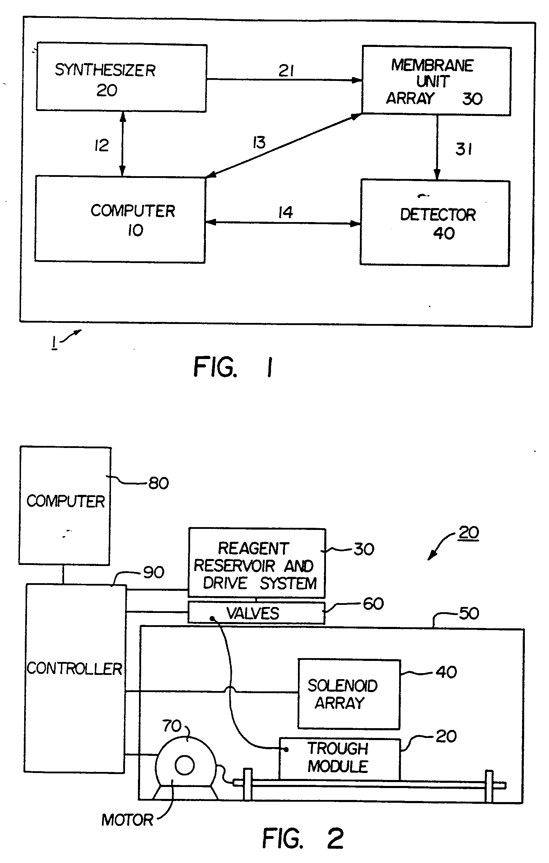

As shown in FIG. 1 and described more fully below, the principal components of automated sequencer 1-1 of this invention are central computer 1-10, synthesizer 1-20, membrane unit array 1-30 and detector 1-40.*Central computer 1-10 not only assembles and analyzes sequence, but also controls and coordinates the operation of all of the other components of automated sequencer 1-1.

Central computer 1-10 constructs nucleic acid sequences by processing descriptions of hybridized sequencing patterns received from detector 1-40. Such processing includes the steps of recording the descriptions of hybridized sequencing patterns, interpreting each such description to construct a portion of a nucleic acid sequence that corresponds to the description, and combining portions of nucleic acid sequences to construct longer nucleic acid sequences. Based on analyses of these constructed sequences, computer 1-10 then predicts a next set of oligomers to be synthesized by synthesizer 1-20, and controls the synthesis of those oligomers.

In conjunction with the prediction and synthesis of oligomers, computer 1-10 also selects a particular membrane unit 14-50, contained in membrane unit array 1-30 for hybridization with each predicted and synthesized oligomer. It will be apparent that hybridization of that next set of oligomers to nucleic acid sequencing patterns immobilized on the selected membranes produces additional sequence descriptions that, in turn, are used to extend or to check the previously determined nucleic acid sequence.

The control signals that computer 1-10 generates are represented generally in FIG. 1 as being transmitted to synthesizer 1-20, membrane unit array 1-30, and detector 1-40 over lines 1-12, 1-13 and 1-14, respectively. Lines 1-12, 1-13 and 1-14 are also used to provide feedback information to computer 1-10 concerning the status of synthesizer 1-20, array 1-30 and detector 1-40. Lines 1-14 also transmits sequence information from detector 1-40 to computer 1-10.

Any or all of lines 1-12, 1-13 and 1-14 may in fact communicate with additional computers, microprocessors or microcontrollers that are integrated with synthesizer 1-20, array 1-30 or detector 1-40 to control the particular operations performed by those devices. Computer 1-10, however, has ultimate control over the operation of each of those devices. The central control provided by computer 1-10 enables these component devices to operate in a coordinated, overlapping manner, thereby performing the series of steps required to construct nucleic acid sequences rapidly, automatically and efficiently. Accordingly, the control provided by computer 1-10 effects a reduction in the total time required to determine the sequence of a nucleic acid molecule.

Synthesizer 1-20 is capable of simultaneously synthesizing a plurality of oligomers of any chosen sequence in response to and in accordance with instructions received from computer 1-10 over line 1-12. By simultaneously synthesizing multiple oligomers, synthesizer 1-20 substantially decreases the time that would otherwise be required to synthesize individual oligomers serially, and decreases the expense and complexity required to network multiple synthesizers, each capable of synthesizing only one oligomer at a time. In addition to synthesizing oligomers from constituent nucleotides, synthesizer 1-20 purifies the synthesized oligomers, labels the purified oligomers appropriately with radioactive or other detectable labels, and provides each labelled oligomer to fluid transportation system 1-21 for transfer to its respective selected membrane for hybridization.

As described above, synthesizer 1-20 is capable of the simultaneous synthesis of a large number of different oligomers. Synthesizer 1-20 comprises a multiplicity of surfaces upon which each of such oligomers can be synthesized. For purposes of illustration, synthesizer 1-20 is described as having six surfaces, although it may incorporate many more surfaces, thus allowing the simultaneous synthesis of many more oligomers.

Synthesizer 1-20 also includes a number of troughs that hold reagents. The reagent troughs are preferably formed within an inert material such as plastic. The directed movement of a surface in and out of a series of reagent troughs allows synthesis of an oligomer of any desired nucleotide sequence.

Synthesizer 1-20 further comprises a system of valves (e.g., 7-80), lines (e.g., 7-70), and reservoirs to

* The first number (i.e., the number preceding the hyphen) refers to the figure number. The second number refers to the particular element of that figure.

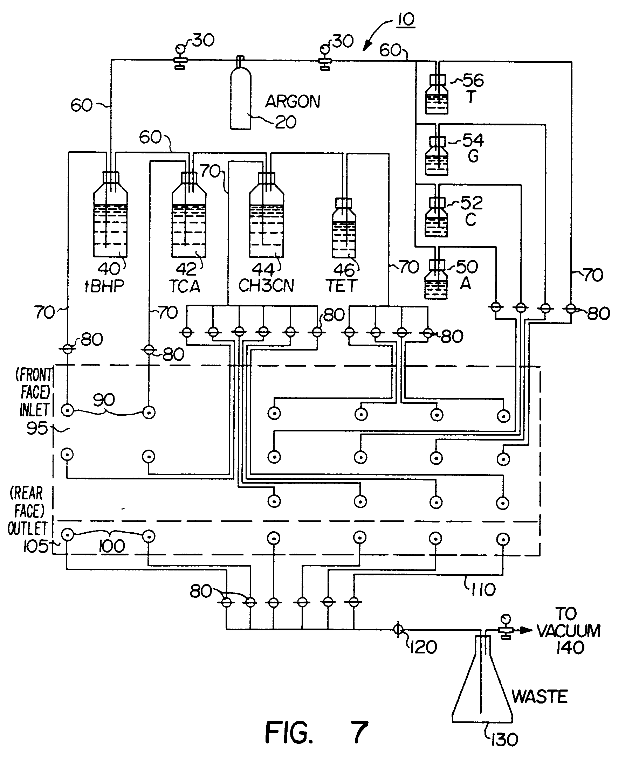

supply the troughs with reagents (e.g., 7-40), and a motor (e.g., 2-70) to move troughs 2-20 (See FIGS. 2 and 7).

Each surface upon which the nascent oligomer will be formed is associated with a solenoid capable of independently raising or lowering that surface into a reagent trough. The solenoids and their associated surfaces are arranged in an array above the reagent troughs. For example, the array may comprise a series of rows of solenoids. The troughs can be moved, for example by a stepper motor, to allow any surface access to any trough. It will be understood that a plurality of surfaces may be simultaneously moved, thus allowing multiple synthesis of like or different oligomers.

The operation of synthesizer 1-20 is automatically controlled by a computer device. The computer device may be computer 1-10 or, preferably, a separate computer, microprocessor or microcontroller associated with synthesizer 1-20. The computer device controls the various operations of synthesizer 1-20, and communicates directly with fluid transportation system 1-21 concerning the availability and location of particular synthesized oligomers. If a separate computer device is used, that device receives instructions from computer 1-10 over line 1-12 as to the sequence of the predicted oligomer to be synthesized and informs computer 1-10 over line 1-12 of the status of synthesizer 1-20, including the status of synthesis of each predicted oligomer.

As shown in FIG. 2 and described below, the principal components of preferred synthesizer 1-20 are trough module 2-20, reagent reservoir and drive system 2-30, solenoid array 2-40, gastight enclosure 2-50, valve block 2-60, motor 2-70, computer 2-80 and controller 2-90. Each is described in detail below.

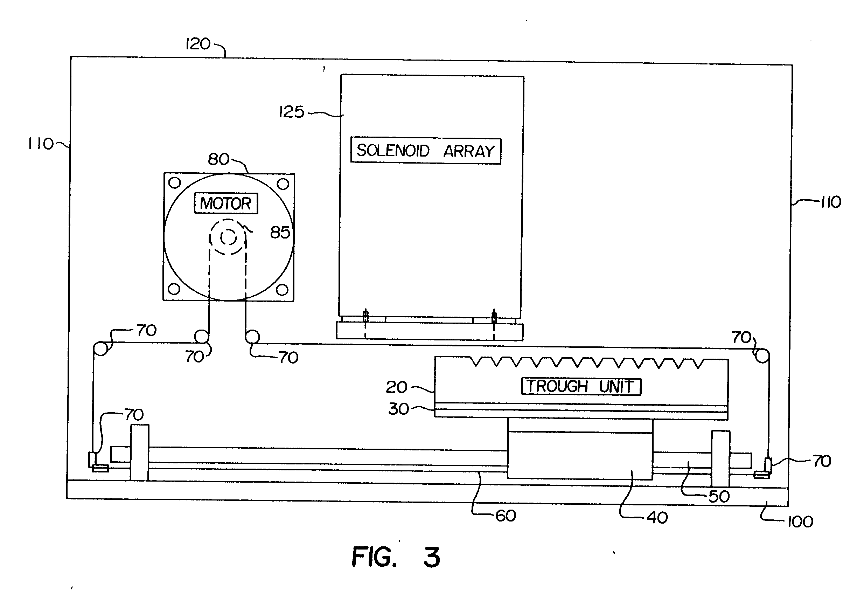

As shown in FIG. 3, the trough assembly includes trough module 3-20,*mounting assembly 3-30, pillow blocks 3-40, travel rails 3-50, drive belt 3-60, pulleys 3-70, stepper motor 3-80, and main drive pulley 3-85.

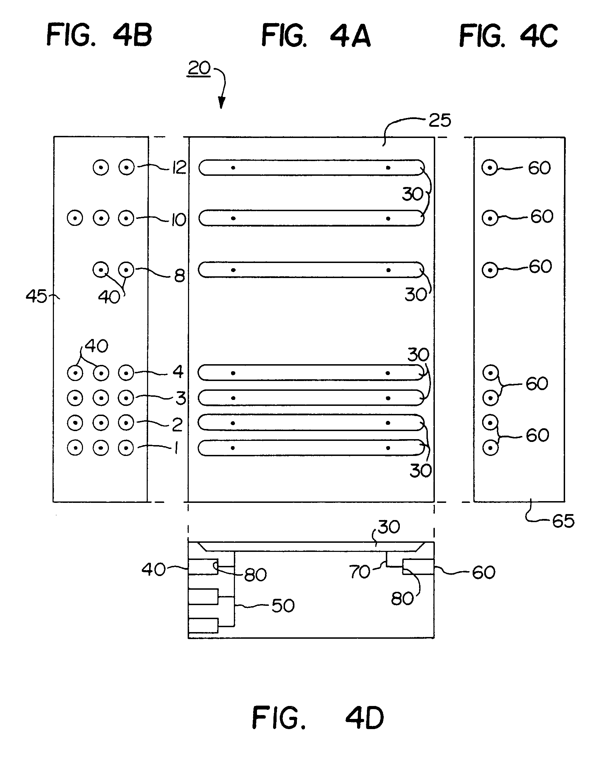

The trough module, shown in FIG. 4, may be milled from a solid block of polypropylene, although any similar inert material is suitable. In the preferred embodiment, the trough module 4-20 (3-20, 2-20) is 5 inches in length, 1.275 inches deep, and 3.375 inches wide. The troughs 4-30, which are formed in top face 4-25 of trough module 4-20, are numbered 4-12, 4-10, 4-8, 4-4, 4-3, 4-2, 4-1. With the exception of trough 4-8, the troughs are preferably milled to a 60° angle at the base and are 5mm deep. Trough 4-8 may be round bottomed and 8mm deep to facilitate a higher volume of reagent flow during the detritylation step of oligomer synthesis. Troughs 4-30 may be spaced 9mm on-center, so as to be compatible with the spacing found in commercially available 96 well laboratory dishes. Reagent inlet ports 4-40, located on front face 4-45 of trough module 4-20, and reagent delivery lines 4-50 allow delivery of reagents to the troughs 4-30. Reagent inlet ports 4-40 may be tapped for standard 1/4" 28 connections. Reagent exhaust ports 4-60, located on back face 4-65 of trough module 4-20, and reagent exhaust lines 4-70 allow removal of reagents from troughs 4-30. Reagent exhaust ports 4-60 may be tapped for standard 1/4" 28 connections.

As shown in FIG. 4D, bottom surface 4-80 of the reagent inlet and exhaust ports is preferably flat, to allow a good seal with standard 1/4" 28 connectors. All ports and lines are preferably constructed so as to minimize the dead volume of the system while still allowing adequate flow rates. Their surfaces should be smooth to facilitate washing and to minimize contamination.

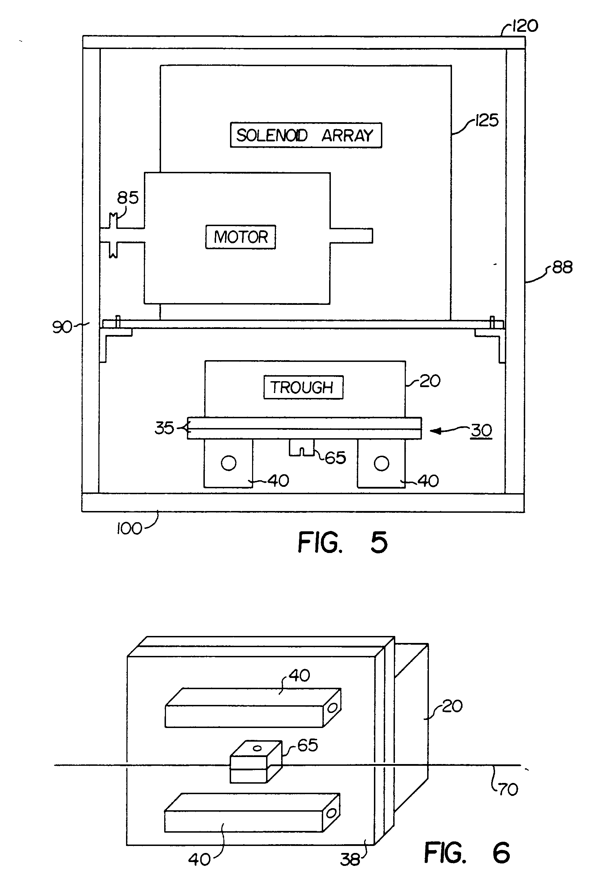

In the preferred embodiment shown in FIGS. 3, 5, and 6, trough module 3-20 is mounted on pillow blocks, (for example TWN-4-ADJ super ball bushing twin pillow block, Atlantic/Tracy, Inc.) to allow travel of trough module 5-20 (3-20) on travel rails 3-50. Mounting assembly 5-30 (3-30) includes two 1/8 inch aluminum plates 5-35 to which trough module 5-20 (3-20) is rigidly but removably mounted. Pillow blocks 5-40 (3-40) are mounted on the underside of the mounting assembly 5-30 (3-30). Travel rails 3-50 pass through pillow blocks 3-40 (5-40). Drive belt 3-60 is fixed rigidly to drive belt anchor 5-65 and is held in position by pulleys 3-70. Angular displacement of main drive pulley 3-85 by stepper motor 3-80 is translated by drive belt 3-60 into transverse displacement, along travel rails 3-50, of trough module 3-20.

Main drive pulley 3-85 is preferably located as close to back plate 5-90 of the gas tight enclosure as possible. Drive belt 3-60 is preferably routed so that it will not interfere with the solenoid array, the reagent supply or exhaust lines connected to the trough unit, or other elements of the device. Drive belt 3-60 is positioned as closely to the ends of the gas-tight enclosure as possible and as close to base plate 3-100 (5-100) as possible, and is routed along the centerline of the baseplate 5-100 to trough module 5-20. Kill switches (not shown) may be appropriately placed to limit the travel of trough module 3-20 to prevent it from moving into glass endplates 3-110.

The reagent reservoir and drive system provides for the supply and removal of reagents, e.g., monomer, oxidizer, and rinsing agents, to the troughs.

The preferred embodiment is shown in FIG. 7. Reagent reservoir and drive system 7-10 includes gas

* The same element may have different numbers on different figures (e.g., the trough module is 2-20, 3-20, 4-20 and 5-20).

source 7-20, metering valves 7-30, reagent reservoirs 7-40, 7-42, 7-44 and 7-46, monomer reservoirs 7-50, 7-52, 7-54, and 7-56, gas lines 7-60, delivery lines 7-70, two-way valves 7-80, reagent inlet connectors 7-90 (connected to the front face 7-95 of the trough module), reagent exhaust connectors 7-100 (connected to the backface 7-105 of the trough module), exhaust lines 7-110, three way valve 7-120, vacuum trap 7-130, and vacuum source 7-140.

In the preferred embodiment, gas pressure from gas source 7-20 provides the motive force for the transfer of reagents from their reservoirs 7-40, 7-42, 7-44 and 7-46, through delivery lines 7-70, to reagent inlet connectors 7-90, and thus to the troughs. Motive force for the removal of reagent or monomer from the troughs is provided by vacuum source 7-140. The delivery or removal of a reagent is controlled by opening and closing appropriate subsets of valves 7-80 or 7-120. The operation of valves 7-80 and 7-120 is controlled by computer. An example of such control is described below.

Argon is a suitable inert gas (although other inert gases may also be suitable) for use in the reagent reservoir and drive system 7-10. A standard 2-stage regulator may be used to release ultra-pure argon from the supply tank, for instance, at approximately 10 psi. The lines supplying argon to the monomer reservoirs may be set at 6 psi. The monomers and tetrazole may be delivered from the bottles they are shipped in. Argon pressure may be delivered to each bottle through a 20 gauge 1 1/2" needle attached to a male luer (w/lock)-1/4" 24 adaptor connected to the appropriate general valve connector. The other reagents are preferably stored in separate reservoirs, for instance, Kontes HPLC/synthesizer reservoirs. Argon may be delivered to these separate reservoirs at, for instance, 3 psi directly to inlet ports in the caps of the reservoirs.

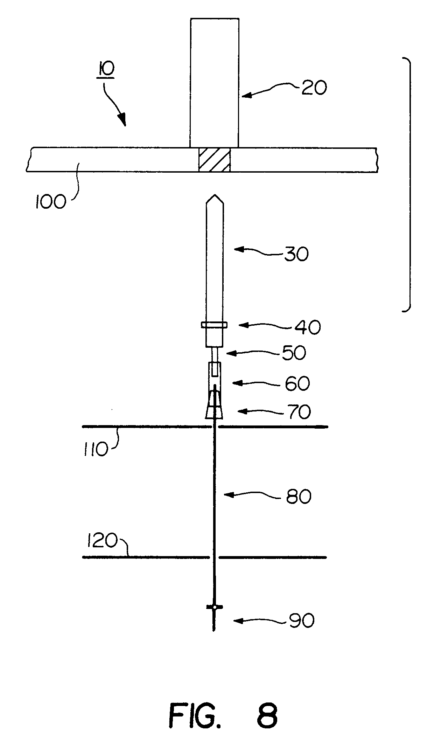

The preferred embodiment includes a solenoid-piston-reagent-pin assembly 8-10, as shown in FIG. 8. The assembly includes solenoid 8-20, piston 8-30, retaining ring 8-40, connecting rod 8-50, adaptor 8-60, pin socket 8-70, pin 8-80, and reagent tip 8-90.

A Guardian T 3.5 X 9-C12D, 12 volt DC continuous duty tubular solenoid is a suitable solenoid. Retaining ring 8-40 may be fabricated from polypropylene, e.g., a one millimeter thick slice cut from a Rainin RT96 pipette tip or any other suitable material. Connecting rod 8-50 is a shaft, preferably plastic, which can be fabricated, for example, from the positive displacement piston of a Gilson CP-250 pipette. Adaptor 8-60 may be fabricated from a piece of flexible tubing, for example, Cole-Parmer 1/32 X 3/32 C-flex tubing. Pin socket 8-70 may be fabricated from the distal six millimeters of a Rainin RT96 pipette tip. Pin 8-80 may be a steel rod and can be fabricated from the displacement piston of a Gilson CP-50 pipette. Reagent tip 8-90 is preferably made of polypropylene. The proximal fitting from the steel displacement piston of a Gilson CP-50 pipette may be used for a reagent tip.

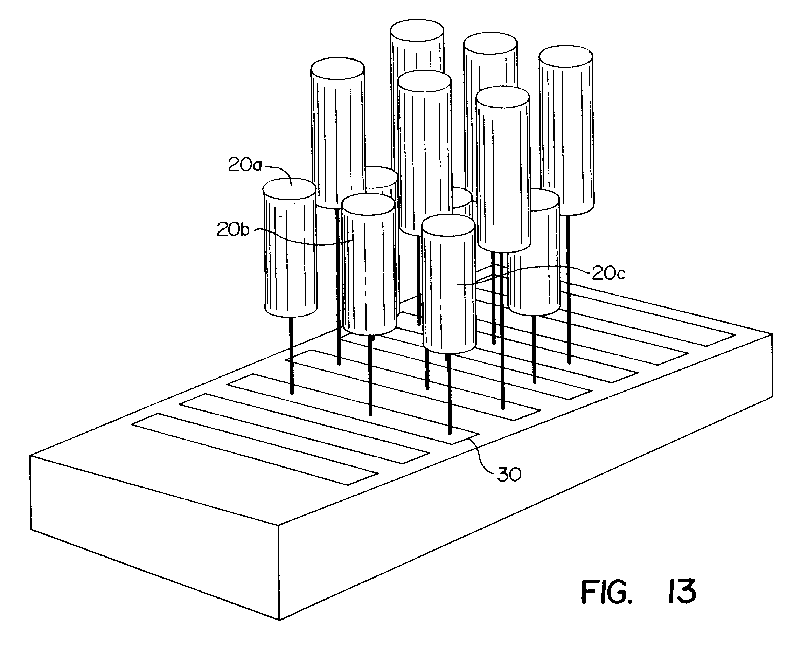

Assemblies 8-10 are mounted in solenoid array frame 8-100. Frame 8-100 may be fabricated from a Rainin RT96 pipette box. Assemblies 8-10 can be positioned in an array such that the longitudinal axis of the pistons are aligned with the spacing found in standard 96-well titre plates. Solenoids 8-20 are preferably positioned in a staggered, two tier, configuration, because the solenoid diameter is too great to allow them to be packed adjacently, i.e., at the same level. (See FIG. 13). The two tier arrangement also allows improved heat dissipation.

In the preferred embodiment, piston 8-30 is inserted into solenoid 8-20. Retaining ring 8-40 is fitted onto piston 8-30. Connecting rod 8-50 links adapter 8-60 to piston 8-30 and on the other end to pin socket 8-70. Pin 8-80 fits removably into pin socket 8-70. Retaining ring 8-40 prevents the piston from seating fully on energizing of solenoid 8-20. If allowed to seat fully residual magnetism prevents the piston from descending when the power is removed. Beneath the piston assembly, upper guide plate 8-110 and lower guide plate 8-120 align reagent pin 8-80 and direct it to the trough. Guideplates 8-110 and 8-120 are fixed rigidly with respect to solenoid array frame 8-100. A clearance of about 1mm exists between tip 8-90 and the top surface of the trough when the solenoids are energized. Upper guide plate 8-110 prevents the piston from falling out of the solenoid and determines the magnitude of piston travel. Total piston travel is about 8mm.

Oligomers are synthesized on a solid phase substrate surface adhered to the reagent tips. Any suitable solid phase substrate surface, including glass fiber, cellulose, or controlled pore glass beads, can be used to form the surface. Commercially available controlled pore derivatized beads to which A, T, G, or C monomer has been attached such as used in column based DNA synthesis are particularly convenient for use with the synthesizer. Examples include those available from ABI with a controlled pore glass ABI part number 400386, C controlled pore glass ABI part number 300387, G controlled pore glass ABI part number 400388, and T controlled pore glass ABI part number 400389 or from Milligen.

The beads may be adhered to the reagent tip by heating the reagent tip until it is just molten, then forcing the heated tip into a shallow container filled with the appropriate bead. As understood by those skilled in the art, when beads coupled to a given monomer are used to synthesize a surface, that monomer will provide the first nucleotide, in the 5' to 3' direction, of the oligomer formed on that surface.

Gas-tight enclosure 2-50 provides a solid base on which other components, e.g., motor 2-70, travel rails 3-50, and solenoid array 2-40 can be mounted. Gas-tight enclosure 2-50 also isolates reagent tips 8-90 and reagents from the atmosphere.

The entire enclosure may be purged of atmospheric gases by argon delivered through a regulator.

In the preferred embodiment (FIGS. 2, 3 and 5), gas-tight enclosure 2-50 has front plate 5-88, back plate 5-90, and base plate 5-100, which may be fabricated from 0.3125" aluminum, and endplates 3-110 and top plate 3-120 which may be fabricated from 0.3125" glass. Silicone gaskets are glued to the glass plates, and the glass plates clamped to the aluminum structure to provide a gas-tight environment. A humidity meter (not shown) may be attached to top plate 3-120 with its probe snaked between the aluminum side and top silicone seal. Lines 7-70 delivering fresh reagent enter the device through the front plate 5-88. Exhaust lines 7-110, argon purge lines (not shown), and electrical connections, pass through back plate 5-90. Motor 2-70 is attached to back plate 5-90. Trough module 3-20 travels on rails 3-50 that are attached to base plate 3-100 (5-100). Solenoid array 3-125 (5-125) rests on supports that are connected to front plate 5-88 and back plate 5-90. Solenoid array 3-125 (5-125) is firmly seated yet removable.

The valve block provides valves that control the flow of reagents to and from the reagent troughs.

In the preferred embodiment, the valve block consists of 24 12VDC 2-way teflon valves 7-80 (e.g., GV#2-17-900, General Valve Corp.) that control the flow of monomers and other agents to and from the trough module and 3-way teflon valve 7-120 CA (e.g., GV#1-17-900 General Valve Corp.) that controls exhaust vacuum. 2-way valves 7-80 are closed when not energized and open when energized. All 2-way exhaust valves 7-80, (e.g., Omega relay # 7-12, see below) are preferably connected to 3-way valve 7-120 such that the main force of the vacuum is isolated from 2-way valves 7-80 when 2-way valves 7-80 are closed. The vacuum valves 7-80 and 7-120 may be electrically isolated from each other via diodes.

The valves may be mounted on Rainin RT96 pipet tip racks and housed in Rainin TR96 pipet tip boxes. All non-monomer connections may be made with 1/16" x 1/32" teflon tubing and the appropriate fittings supplied by, for example, General Valve according to their recommendations. The monomers and tetrazole lines may consist of 0.007" ID tubing. Although small-bore tubing is desirable in that it minimizes dead volume, the additional trough-filling time required by small bore tubing may be undesirable.

A Super Vexta PH268M-E1.5B 2-phase stepper motor, available from Inductive Components, is a suitable motor for moving trough module 3-20.

If the synthesizer is controlled by a separate computer device, either Digital Microvax or Radio Shack TRS 80 Model 100 computers are suitable. Instructions from computer 1-10, or the separate computer device, determine the sequence in which the reagents come into contact with each surface and thus determine the nucleotide sequence of the oligomer synthesized on each surface. Such instructions include instructions: to open or to close various valves, to introduce or to remove a reagent from a trough, to move the troughs, or to raise or to lower a given surface. The instructions are implemented by an interface.

A suitable interface is Omega OM900 Series Interface (Omega Instruments, Stanford, Connecticut) or similar device capable of converting instructions from computer 1-10, or a separate computer device, into signals that can control the electro-mechanical devices of synthesizer 1-20. The Omega OM900 Series interface includes a central processing unit (OM992 Central Processing Unit), an interface power supply (OM-903 Power Supply), and a multiplex module (OM915 Multiplex Module) with 32 individually addressable relays which are used to control synthesizer 1-20. For example, if six solenoids are included in synthesizer 1-20, the 32 relays may be assigned as shown in Table 1.

## Table 1

| Relay Number | Function controlled |
|---|---|
| 1 | Oxidizer reagent (tert-butyl hydroperoxide (tBMP) delivery valve |
| 2 | Detritylation reagent (trichloroacetic acid (TCA)) delivery valve |
| 3 | Adenine ($B_z$ dA cyanoethyl phosphoramidite) delivery valve |
| 3 | Tetrazole delivery valve |
| 4 | Cytosine ($B_z$ dC cyanoethyl phosphoramidite) delivery valve |
| 4 | Tetrazole delivery valve |
| 5 | Guanine (iBu dG cyanoethyl phosphoramidite) delivery valve |
| 5 | Tetrazole delivery valve |
| 6 | Thymine (T cyanoethyl phosphoramidite) delivery valve |
| 6 | Tetrazole delivery valve |
| 7 | Oxidizer (tBHP) reagent exhaust valve |
| 8 | Detritylation (TCP) reagent exhaust valve |
| 9 | Adenine exhaust valve |
| 10 | Cytosine exhaust valve |
| 11 | Guanine exhaust valve |
| 12 | Thymine exhaust valve |
| 13 | Oxidizer rinse delivery valve |
| 14 | Detritylation rinse delivery valve |

| | |
|---|---|
| 15 | Adenine rinse delivery valve |
| 16 | Cytosine rinse delivery valve |
| 17 | Guanine rinse delivery valve |
| 18 | Thymine rinse delivery valve |
| 19 | Solenoid No. 1 (initial activation), 12 VDC supply |
| 20 | Solenoid No. 2 (initial activation), 12 VDC supply |
| 21 | Solenoid No. 3 (initial activation), 12 VDC supply |
| 22 | Solenoid No. 4 (initial activation), 12 VDC supply |
| 23 | Solenoid No. 5 (initial activation), 12 VDC supply |
| 24 | Solenoid No. 6 (initial activation), 12 VDC supply |
| 25 | Stepper motor (motor drive) 5 VDC supply |
| 26 | Stepper motor (motor direction) 5 VDC supply |
| 27 | Solenoid No. 1 (maintenance), 5 VDC supply |
| 28 | Solenoid No. 2 (maintenance), 5 VDC supply |
| 29 | Solenoid No. 3 (maintenance), 5 VDC supply |
| 30 | Solenoid No. 4 (maintenance), 5 VDC supply |
| 31 | Solenoid No. 5 (maintenance), 5 VDC supply |
| 32 | Solenoid No. 6 (maintenance), 5 VDC supply |

Solenoids Nos. 1 to 6, described in Table 1 above, are each associated with a corresponding solenoid-piston-reagent-pin assembly.

For such a configuration, it will be apparent that Relay No. 1 opens a valve which allows oxidizer to flow into the appropriate trough (trough 4-12); Relay No. 3 opens two valves, one which allows adenine monomer, and one which allows tetrazole to flow into the appropriate trough (trough 4-4); Relay No. 7 opens a valve which allows the contents to be removed from the oxidizer trough (trough 4-12); Relay No. 13 opens a valve which allows a rinsing solution to flow into the oxidizer trough (trough 4-12); Relay No. 19 controls the 12 VDC supply to solenoid No. 1 (12 VDC is required to initially activate the solenoid); Relay No. 27 controls the supply of 5VDC to solenoid No. 1 (the voltage required to maintain the solenoid in the activated state); and Relay Nos. 25 and 26 which control respectively, the power to the stepper motor and the direction of the stepper motor, control the positioning of the trough module.

The assignment of a single relay to control both monomer and tetrazole valves (relays 3 to 6) is due to constraints imposed by the number of relays on the OM-915. Increasing the number of relays would remove the need to place more than one valve on a relay. More importantly, increasing the number of relays would allow an increase in the number oligomers that can be simultaneously synthesized. For example, the system could easily be expanded to seven more OM-915 units. In such a configuration, one OM-915 unit would be dedicated to valve and motor control and the other OM-915 units would be dedicated to solenoid control. The use of seven OM-915 units thus configured would allow 96 controllable solenoids, and thus allow the synthesis of 96 oligomers simultaneously.

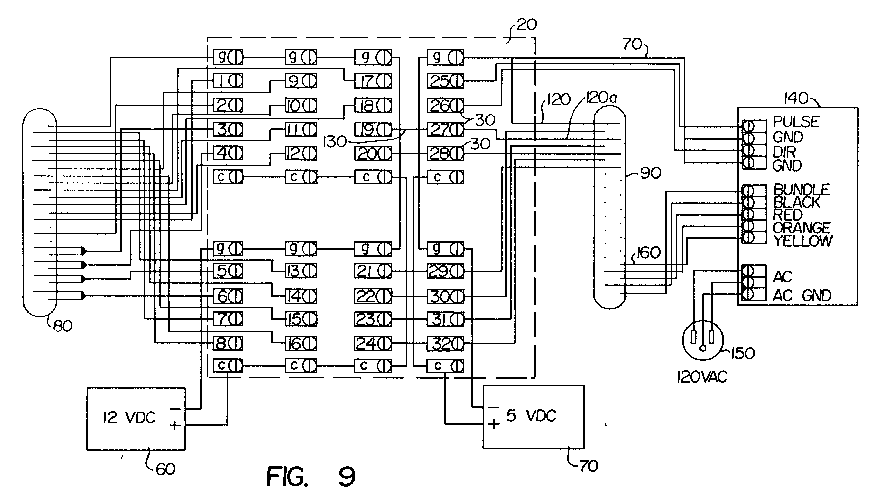

FIG. 9 depicts the terminal board 9-20 of the OM-915 interface and associated connectors. Relay terminals 9-30 are labeled with their relay numbers. Power is supplied to relays 9-30, in banks of four relays, (e.g., Relay Nos. 1 to 4 constitute a bank, and Relay Nos. 5 to 8, constitute a separate bank), by common contact (labeled c) and ground contact (labeled g). Relay Nos. 1 to 24, which control valves, are supplied with the 12 VDC from 12 VDC source 9-60. Relay Nos. 25 and 26, which control the motor, are supplied with 5 VDC from 5 VDC source 9-70. Relay Nos. 27 to 32, which control the activation and maintenance of the energization of the solenoids, are supplied with 12 VDC (for initial activation) from Relay Nos. 19 to 24, and 5 VDC (for maintenance of activation) from the 5 VDC source 70. A SOLV 30-12 12 VDC 4A power supply (Newark Electronics), is a suitable 12 VDC source and an Elpac Model WM113 + 12/-12/ + 5 VDC power supply is a suitable 5 VDC source.

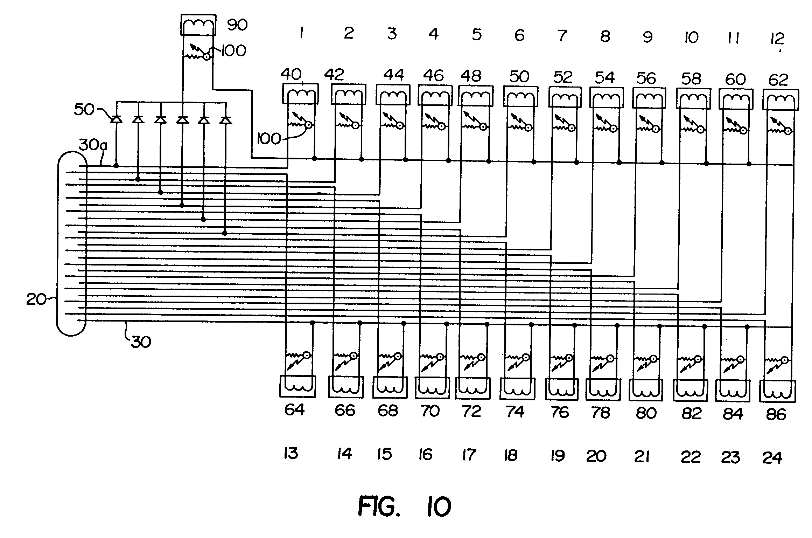

Relays controlling the valves are connected to controller-side valve block connector 9-80. As described in Table 1, Relay Nos. 3, 4, 5, and 6 each controls two valves. Each of these relays is thus connected to two pins on controller-side valve block connector 9-80. Controller-side valve block connecter 9-80 is connected to machine-side valve block connector 10-20 shown in FIG. 10 which depicts the wiring of the valve block.

Machine-side valve block connector 10-20 may be connected by lines 10-30 to two-way valve controlling solenoids 10-40 to 10-86 and three-way valve controlling solenoid 10-90. The valve solenoids may be electrically isolated by diodes 10-50 or light emitting diode: 10-100.

Activation of any of valve controlling solenoids 10-40, 10-42, 10-44, 10-46, 10-48, or 10-50, which are the valves controlling exhaust of a reagent from a trough, also preferably activates 3-way valve controlling solenoid 10-90. For example, activation of valve controlling solenoid 10-40 by a signal transmitted on line 10-30a also activates the 3-way valve controlling solenoid 10-90. Thus, both the exhaust valve and vacuum control valve are opened, allowing vacuum to exhaust the trough. The circled numbers in FIG. 10 indicate the number associated with the 2-way valve controlled by a solenoid.

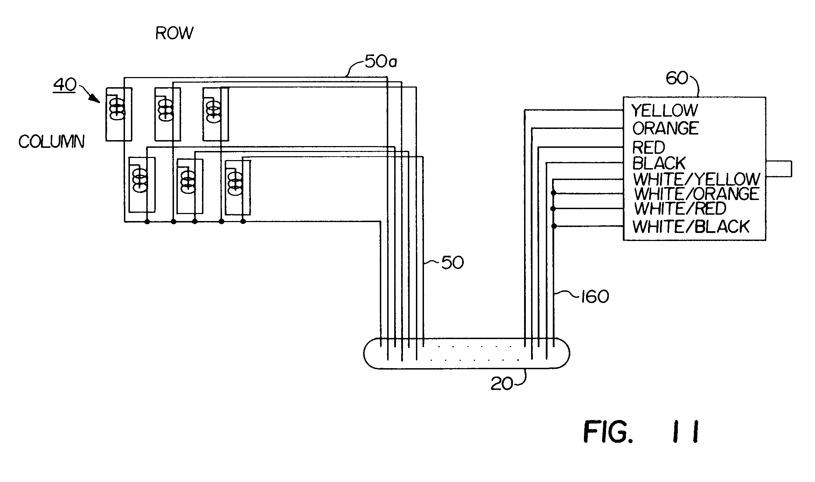

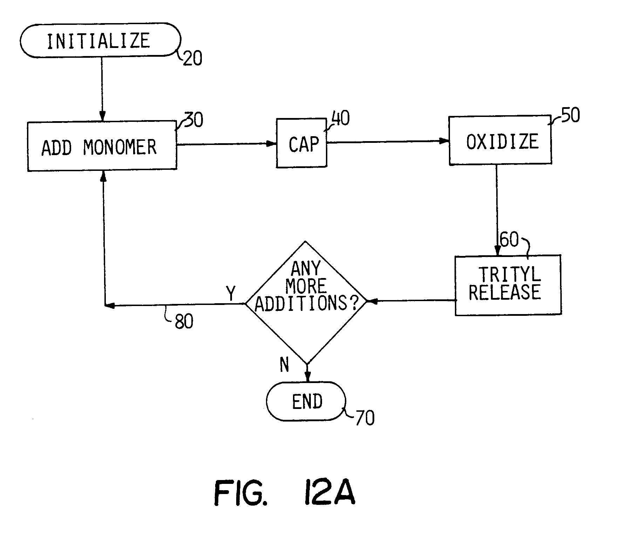

As shown in FIGS. 9 and 11, Relay Nos. 25 to 26, which control stepper motor 11-60, are preferably connected directly to stepper motor controller 9-140 by lines 11-160. 120 VAC source 9-150 is also connected to stepper motor controller 9-140. Lines 9-160 connect stepper motor controller 9-140, by way of controller-side enclosure connector 9-90, machine-side enclosure connector 10-20, and lines 9-160, to stepper motor 11-60. Relay Nos. 19 to 24 and 27 to 32, which control the solenoids, are connected by lines 9-120 to controller-side enclosure connector 9-90. Controller-side enclosure connector 9-90 connects to machine-side enclosure connector 11-20 shown in FIG. 11. Machine-side enclosure connector 11-20 is connected to solenoids 11-40, which may be organized in a 2 x 3 array, by lines 11-50.

With reference to Table 1, and FIGS. 9 and 11, a solenoid is preferably activated as follows. A solenoid is lifted by 12 VDC. For example, a signal from the computer closes Relay No. 19, supplying 12 VDC by line 9-130 to Relay No. 27. Relay No. 27 in turn supplies 12 VDC by line 9-120a to controller-side enclosure controller 9-90, to machine-side enclosure 11-20, to line 11-50a and, to hence, to solenoid 1.

After being lifted, the solenoid may be maintained in the activated state by 5 VDC. The computer instructs Relay No. 27 to close, supplying 5 VDC to solenoid 1 and opens Relay No. 19, to remove the 12 VDC potential used to initially activate solenoid 1. At another signal from the computer, Relay No. 27 opens and the 5 VDC potential is cut off.

The simultaneous synthesis of multiple oligomers of any chosen sequence is directed by computer 1-10. The positioning of the troughs, movement of the surfaces, and the filling and emptying of the troughs may be controlled by computer 1-10 alone or, preferably, by a separate computer system associated with synthesizer 1-20.

11

For example, where a separate computer system is used, it may operate using a Fortran program (Appendix A, attached as part of the description).

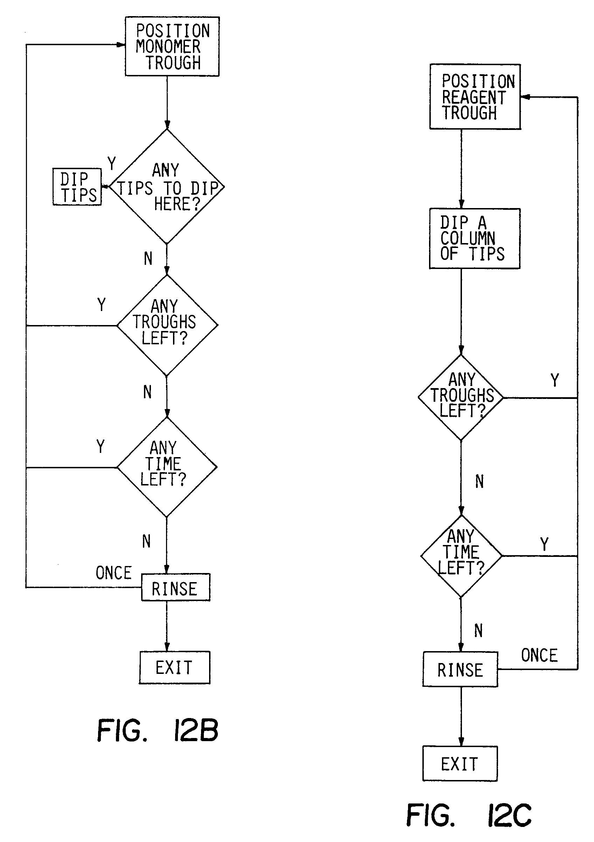

The general operation of a suitable program is shown in FIG. 12. As shown in FIG. 12A the program first initializes the system (step 12-20). Initialization may include filling the reagent troughs, bringing selected monomer troughs into position under appropriate solenoids, then dipping the surfaces into any trough required for activation.

A monomer is then added (step 12-30), the growing molecules are capped (step 12-40), oxidized (step 12-50) and deprotected (step 12-60). If there are no more additions, the program ends 12-70. If there are more monomers to be added (step 12-80), the program returns to add the next monomer (step 12-30).

The process for the addition of a monomer is described in more detail in FIG. 12B. The process for capping, oxidation, and detritylation is described in more detail in FIG. 11C.

The program may also include a subroutine that, upon entry of the predicted sequences, determines the optimum assignment of sequences to surfaces; determines the optimum order of the placement of reagent troughs; or controls post-synthesis modification, such as elution or washing.

The chemistry of DNA synthesis is well known to those skilled in the art, see e.g., Froeler et al. 1988, Nuc. Acid. Res. 14:5399-5407, hereby incorporated by reference. The phosphoramidite method, see Matteucci et al., 1981, J. Am. Chem. Soc. 103:3185-3191, hereby incorporated by reference and Caruthers et al., 1987, Methods Enzym. 154:287-313, hereby incorporated by reference, is suitable for use with synthesizer 20 and is described briefly below.

In the phosphoramidite method, 5' protected monomers are added to the 5' OH group on the growing molecule chain. Addition of a monomer subunit to the growing nucleotide chain requires the following steps: (1) removal of the dimethoxytrityl protecting group from the previously added (or starting) monomer with trichloroacetic acid (TCA) to form a reactive 5' hydroxyl group; (2) the addition of a 5' dimethoxytrityl protected monomer by condensation; (3) acetylation or capping of the unreactive deoxynucleoside (Step 3 is optional); and (4) oxidation of the phosphite triester to the phosphate triester with tert-butyl hydroperoxide or iodine/water. Synthesis proceeds stepwise in a 5' to 3' direction by the sequential addition of monomers.

Oligomers are synthesized on the surfaces that are adhered to the reagent tips. The reactions needed to add a monomer to the nascent oligomer synthesized on a surface are effected by: (1) utilizing troughs filled with the appropriate reagents, such as a monomer, oxidizer, or rinsing agent, (2) positioning the appropriate troughs beneath the appropriate surfaces in an appropriate order, and (3) lowering the surface into the trough. For example, addition of a monomer to a growing chain on a surface requires the following steps: (1) activating the previously added monomer by positioning the trough filled with detritylation reagent below the surface, dipping the surface into the detritylation reagent to remove the dimethoxyl-trityl protecting group, removing the detritylation reagent from the trough, filling the trough with a rinsing agent such as, acetonitrile to rinse the trough and surface, and lifting the surface; (2) adding a protected monomer by repositioning a trough containing the appropriate monomer below the surface, dipping the surface into the monomer trough, removing the monomer reagent from the trough, filling the trough with rinsing agent to rinse the trough and surface, and lifting the surface; and (3) oxidizing the phosphite triester bond by positioning a trough containing the oxidizer, such as tert-butyl hydroperoxide under the surface, dipping the surface into the oxidizer to oxidize the phosphite triester bond to a phosphate triester, removing the oxidizer and filling the trough with rinsing agent to rinse the trough and surface, and lifting the surface from the trough. This sequence of reactions results in the addition of a protected monomer to the oligomers being synthesized on the surface.

In the specific and particular embodiment described herein, reagents, reaction times, and trough designations are as follows. Adenine monomer reagent consists of one gram of Bz dA cyanoethyl phosphoramidite in 12 ml anhydrous acetonitrile and occupies trough No. 4-4, thymine monomer reagent consists of one gram of T cyanoethyl phosphoramidite in 12 ml anhydrous acetonitrile and occupies trough No. 4-1, cytosine monomer reagent consists of one gram of Bz dC cyanoethyl phosphoramidite in 12 ml anhydrous acetonitrile and occupies trough No. 4-3, and guanine monomer reagent consists of one gram of iBu dG cyanoethyl phosphoramidite in 12 ml anhydrous acetonitrile and occupies trough No. 4-2. Surfaces are exposed to monomer for three minutes then washed in acetonitrile for one minute. It should, however, be understood that other concentrations, times, washes, washing schedules and trough arrangements are within the scope of this invention.

Detritylation reagent consists of trichloroacetic acid in dichloromethane and occupies trough No. 4-8. Surfaces are held in the detritylation reagent for three minutes. The rinsing agent which follows detritylation consists of acetonitrile. Surfaces are rinsed for one minute. The oxidizing reagent consists of tert-butyl hydroperoxide 1.1 M in anhydrous dichloromethane and occupies trough No. 4-12. Surfaces are held in the oxidizing reagent for one minute. After oxidation, the surfaces and troughs are washed in acetonitrile for one minute.

Rinsing normally occurs in the same trough in which the previous reaction occurred, e.g., the post tritylation rinse occurs in trough 4-8.

At the beginning of the run, the enclosure and all lines are purged with argon and all troughs are filled with the appropriate reagents. All surfaces are lifted and the trough module is moved to the initiation position. The initiation position places trough 4-8 (TCA) beneath the designated solenoid.

For the sake of simplicity, the action of a single surface has been described above, in actual operation, synthesis of multiple oligomers will proceed simultaneously on multiple surfaces. This may be accomplished by positioning the solenoids in an array, for instance, an array of rows. For example, as shown in FIG. 13 the solenoids 13-20a, 13-20b, 13-20c, in a given row 13-20a are aligned such that the surfaces can all be dipped into trough 13-30 positioned under that row of solenoids. When a trough is positioned under a row, all of the surfaces in that row that require contact with the reagent in the trough may be lowered simultaneously. Surfaces that do not require contact with the reagent are not lowered. While surfaces in a row above a given trough are being lowered, the solenoids in the adjacent rows in either direction can be lowered in the corresponding adjacent troughs. Thus, synthesis of the oligomers on all surfaces is truly simultaneous.

In addition to synthesizing oligomers, synthesizer 1-20 performs other steps necessary to prepare the synthesized oligomers for use in hybridization. These steps include eluting the oligomers from the surfaces on reagent tips 8-90, purifying the eluted oligomers, labelling the purified oligomers, and placing each labelled oligomer in transportation system 1-21 to be sent to the corresponding selected membrane unit 14-50 (15-50, 16-50, 17-50 and 19-50) contained in membrane unit array 1-30 for hybridization to nucleic acid sequencing patterns bound to membrane 16-51.

It will be obvious to one skilled in the art that the composition of the eluting solution will depend on the mode of synthesis of the oligomers. For example, if conventional oligonucleotide synthesis involving addition of acetate caps onto unreacted nucleotide chains is employed, the longest resulting oligomer will contain a trityl group on the 5' end. Such an oligomer may be eluted and deblocked in one step, for example, by heating in a solution of $NH_4OH$ contained within the well of a 96 well plate. After evaporation of the $NH_4OH$, the oligomers may be labelled directly in the well. The oligomers may then be transferred from the well by fluid transportation system 1-21 to membrane unit array 1-30.

Alternatively, the oligomers may, if desired, be purified to eliminate all but the longest nucleotide chains. Purification may be performed by eluting the oligomers with methanol and automatically transferring the eluted oligomers contained in the wells to a corresponding number of affinity columns, each containing a substance, such as oligopak or Nensorb, that binds the trityl group of the oligomer. Such chromatography thereby removes any capped failure molecules. The purified oligomers are then eluted from the columns into the wells of another disposable multi-well plate.

The oligomers may be labelled in the wells by one of a variety of methods to permit their detection after hybridization to sequencing patterns bound to membrane 16-51. Any of a number of labelling methods may be employed, including those employing radioactive labels [T. Maniatis et al., Molecular Cloning, A Laboratory Manual, Cold Spring Harbor Laboratories, 1982], fluorescent [(L.M. Smith et al., Nuc. Acid. Res., 13, pp. 2399-412 (1985); J.M. Prober et al., Science, 238, pp. 336-41 (1987); W. Ansorge, et al., Nuc. Acid. Res., 15, 4593-603 (1987); J.A. Brumbaugh, et al., Proc. Natl. Acad. Sci. USA, 85, pp. 5610-14 (1988)], calorimetric [(S. Beck, Anal. Biochem., 164, pp. 514-20 (1987); P. Richterich, et al. Biotechniques, 7, pp. 52-59 (1989)], and chemiluminescent labels [S. Beck, et al., Nuc. Acid. Res., 17, pp. 5115-122 (1989)]. As will be understood by one skilled in the art, the method of labelling chosen must conform to the type of detector 1-40 employed. After labelling, any unincorporated label may be removed if desired, by passing the labeled oligomer over a sizing column. It is preferred that the labelling be conducted by a method that does not generate a significant amount of unincorporated label. The labelled oligomers are then introduced into fluid transportation system 1-21, described below, which connects synthesizer 1-20 to each membrane unit 1-50 contained in membrane unit array 1-30.

The above described mechanical steps performed by synthesizer 1-20 after oligomers have been synthesized can be automated using any of a number of commercially available devices, with minor modifications. For example, robots such as the Xymark free-hand robot, and machines capable of handling the multiple-well plates and transferring materials to and from the plates, such as those sold by Biomek and Lab Systems, are readily available for use with synthesizer 1-20.

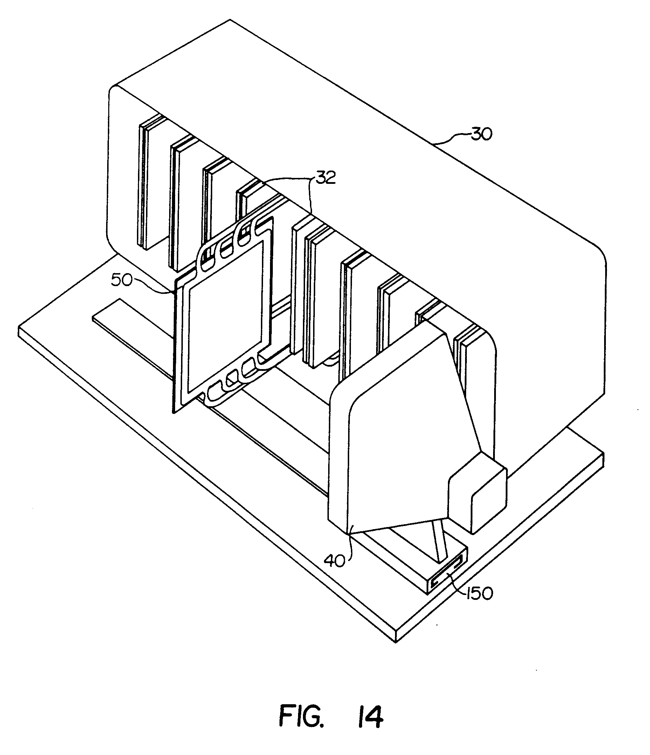

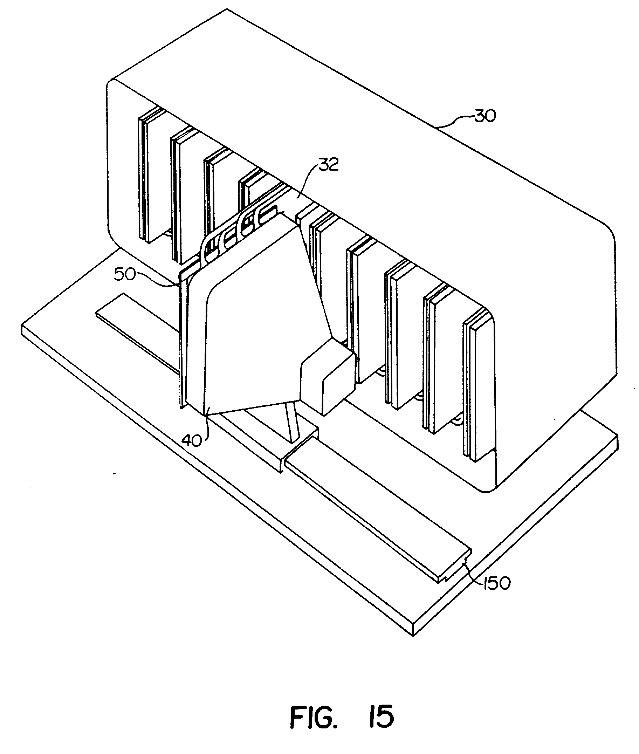

Membrane unit array 1-30, which is shown generally in FIGS. 14 and 15, contains a plurality of membrane unit stations 14-32, each of which holds a membrane unit 14-50. Stations 14-32 are separately connected to transportation system 1-21, enabling fluids to be independently supplied to and removed from each membrane unit 14-50. Array 1-30 may contain any number of membrane units 14-50, with a corresponding number of membrane unit stations 14-32. For example, array 1-30 may contain 400 membrane units 14-50 and stations 14-32. It is apparent that array 1-30 of such size has substantially more stations 15-32 than are shown generally in FIGS. 14 and 15.

Each membrane unit 16-50 (14-50) includes a membrane 16-51, upon which, prior to automated operation of sequencer 1-1, nucleic acid sequencing patterns have been immobilized.

In the preferred embodiment, the patterns bound to membrane 16-51 are multiplex sequencing patterns, i.e., multiple, overlayed, nucleic acid sequence patterns capable of being repeatedly hybridized with different probes to reveal different nucleic acid sequence ladders (see Ohara et al, supra).

The DNA used to generate the multiplex sequencing patterns may be cloned. Alternatively, genomic DNA may be used directly. When using the method and apparatus of the present invention to sequence DNA from higher organisms, for instance chromosomes with the complexity of mammalian DNA, it may be useful to first clone the DNA into vectors such as yeast artificial chromosomes. Genetic manipulation of yeast artificial chromosomes is well known to those skilled in the art.

Any procedure that cleaves DNA at defined locations and results in well defined 5' and 3' ends may be used to fragment the DNA prior to the sequencing reactions. In the preferred embodiment, a multiplex sequencing pattern is generated from chemical cleavage of DNA fragmented by restriction enzyme digestion. The DNA is first digested to completion with different restriction enzymes or combinations of enzymes, such that the resulting DNA fragments are of a given average size, yet have many different 5' and 3' ends. For example, enzymes that recognize a sequence of 6 bases cut DNA an average of once per 4000 base pairs. Digestions with combinations of such enzymes produce smaller fragments. Varying the combinations results in variation of the 5' and 3' ends of the fragments.

When gels that allow reading of a sequence up to approximately 300 base pairs are utilized, it is preferred that the DNA be digested with combinations of enzymes to produce fragments of 700 to 800 base pairs. When longer gels are utilized, for example gels that allow reading of sequence up to 600 bases, it may be advantageous to choose a combination of enzymes that will result in the DNA being cleaved on average every 1000 base pairs. Alternatively, digestion may be performed with multiple single enzymes that recognize a sequence of fewer bases.

After digestion, each sample is subjected to sequencing reactions to generate multiplex sequencing patterns. For example, chemical cleavage reactions such as the five standard reactions [A.M. Maxam and W. Gilbert, "Sequencing End-Labeled DNA with Base-Specific Chemical Cleavages", Meth. Enzym., 65, pp. 499-560 (1980)] may be employed. Modifications of the standard sequencing reactions, for instance those described in United States Patent No. 4,865,968, may also be employed. Alternatively, the multiplex sequencing patterns may be generated by enzymatic synthesis. It is preferred that 1 to 30 micrograms of DNA be employed for each sequencing reaction.

Sequencing reactions derived from a multiplicity of restriction digested DNA samples may then be loaded onto the same gel for subsequent electrophoresis. For example, a single gel may contain the sequencing products of 18 or more single or combination digests.

The products of the sequencing reactions may be resolved electrophoretically to form numerous overlayed nucleic acid sequence ladders, i.e., multiplex sequencing patterns. At present, denaturing gels of 5-20% acrylamide are commonly used for electrophoretic separation. Such gels may be wedge shaped or of uniform thickness, and may be run using a variety of buffer systems and electrophoretic conditions well known to those skilled in the art.

After electrophoresis, the multiplex sequencing patterns are transferred onto membrane 16-51. Such transfer may be accomplished by any of a variety of methods. For example, the patterns may be electrotransferred after electrophoresis as described in G.M. Church and W. Gilbert, supra. Alternatively, the DNA may be transferred during electrophoresis in the method described by S. Beck and F.M. Pohl, "DNA Sequencing With Direct Blotting Electrophoresis", EMBO J., 3, pp. 2905-2909 (1984). A variety of membranes may be employed in the present invention, for example DBM, DPT, and polyvinylidene fluoride membranes. Nylon membranes are preferred, as they are able to withstand vigorous washing.

After transfer, the sequencing patterns must be immobilized on the membrane such that they will remain bonded throughout repeated cycles of hybridization, washing and stripping. It is preferred that the sequencing patterns are covalently bound to the membrane by UV crosslinking, for instance as described in G.M. Church and W. Gilbert, supra. Other techniques of linking nucleic acids to membranes may also be useful and are well known to those skilled in the art.

After binding is complete, each membrane 16-51 is placed in a separate membrane unit 16-50. Membrane unit 16-50 is designed to permit several operations to be repeatedly performed on membrane 16-51, including hybridization of labelled oligomers to the sequencing patterns bound to membrane 16-51, washing of membrane 16-51, movement of hybridized membrane 16-51 to be detected by detector 15-40, and stripping of membrane 16-51.

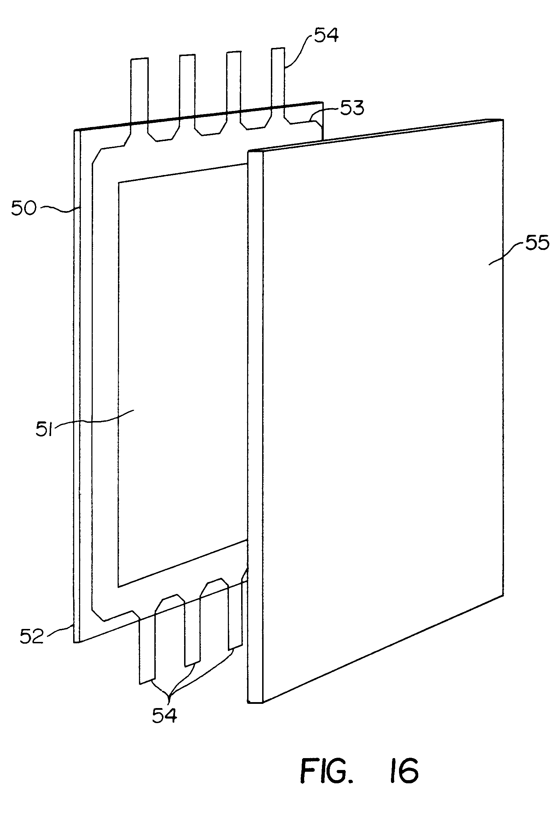

The elements of the preferred embodiment of membrane unit 16-50 are shown in FIG. 16. Membrane 16-51 is bonded to a face of backing plate 16-52 within the margins of plate 16-52. Plate 16-52 may be made of a number of materials including glass or plastic. Flexible sheet 16-53, preferably made of plastic, covers the face of plate 16-52 on which membrane 16-51 is bonded, and is itself bonded to plate 16-52 near the margins of plate 16-52, enclosing a space between sheet 16-53 and membrane 16-51. Flexible sheet 16-53 has at least one, and preferably a series of inlet and outlet tubes 16-54 formed therethrough. The free end of each tube 16-54 is connected to fluid transportation system 1-21 within station 14-32 to supply membrane unit 14-50 (16-50) with fluids and to drain fluids from membrane unit 16-50 for disposal.

Preparation of membranes 16-51 and assembly of membrane units 16-50 preferably occurs prior to the commencement of automated operation of sequencer 1-1. Membranes 16-51 and membrane units 16-50 may be changed, substituted or reassembled during operation of sequencer 1-1.

It will be understood that bonded membrane 16-51 is reusable many times, at least 50 times and probably several hundred times, because the bonding eliminates most of the current sources of failure that are produced through flexing of membranes during hybridization, washing and stripping. By reducing or eliminating the time required to replace failed membranes, the speed of sequencing is much enhanced.

In order for hybridization to occur properly, it is required that flexible sheet 16-53 be separated from membrane 16-51 a specified distance, with fluid contained in the enclosed space between flexible sheet 16-53 and membrane 16-51. It is preferred that the fluid flow between membrane 16-51 and flexible sheet 16-53 be in a 100-300 micron layer.

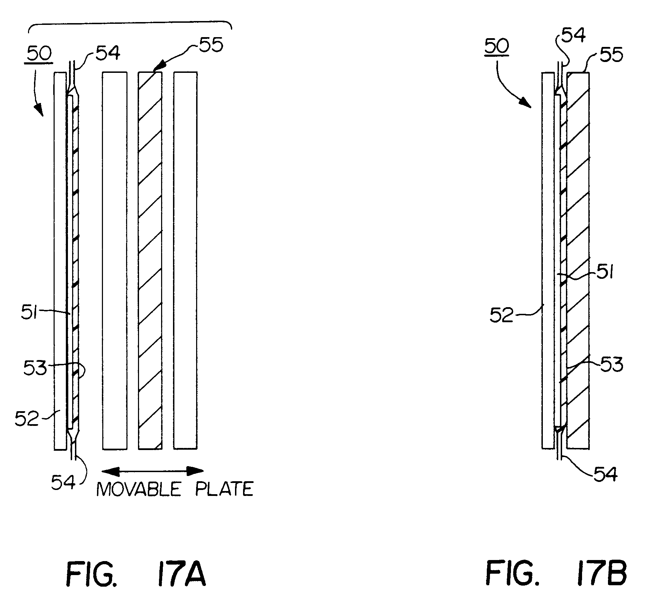

Cover plate 16-55, shown in FIG. 16, is provided for each membrane unit 16-50 to maintain the desired separation between sheet 16-53 and membrane 16-51. Cover plate 16-55 opposes the side of sheet 16-53 facing away from membrane 16-51. The operation of membrane unit 16-50 in conjunction with cover plate 16-55 is depicted in FIGS. 17a and 17b. Cover plate 17-55 (16-55) moves toward membrane unit 17-50 (16-50) from the released position shown in FIG. 17a to the compressed position shown in FIG. 17b. Alternatively, cover plate 17-55 can be fixed and membrane unit 17-50 may be moved, or both cover plate 17-55 and membrane unit 17-50 can moved. In the compressed position, cover plate 17-55 contacts and compresses covering sheet 17-53 to limit pressurized fluid flow to a layer of desired thickness. When so compressed, hybridization, washing and stripping occur.

The movement of cover plate 17-55 between a released position and a compressed position can be achieved by any of a number of well-known devices, and is automatically achieved through computer control. Although the various operations associated with hybridizing, washing and stripping membrane 17-51 within membrane unit 17-50 are ultimately controlled under direction of computer 1-10, another computer, microprocessor or microcontroller associated with array 1-30 may further direct and control the specific operations that are performed in array 1-30. Communication between array 1-30 and computer 1-10, or between a separate computer device associated with array 1-30 and computer 1-10, occurs over line 1-13. Such communication from computer 1-10 informs array 1-30 of the selected membrane unit that each synthesized oligomer will be directed to, and prepares that unit for hybridization. In return, computer 1-10 is informed of the status and availability of each membrane unit contained in array 1-30.

Optimal performance conditions may require regulation of the temperature of the pressurized fluid. Accordingly, in the preferred embodiment, cover plate 17-55 also contains a means to regulate the temperature of the fluid flowing over membrane 17-51. This means is preferably a Peltier effect, but could also be achieved by fluid flow in coils embedded in or backing plate 17-55, where plate 17-55 is made of a thermally conductive material such as a metal. The regulation of temperature is controlled by the separate computer device associated with membrane unit array 1-30 under the direction of computer 1-10, or directly by computer 1-10.

Membrane unit array 1-30 may be of arbitrary size. For example, it may contain 100 units, or more than 1000 units. Each membrane unit 1-50 operates independently of the other units, however. It will thus be understood that, at any given time, any hybridizing, washing or stripping step, or any stage of such step may occur within any membrane unit 17-50. Accordingly, the hybridization process may proceed simultaneously within different membrane units 17-50. Moreover, different oligomers may be used for hybridization within different units 17-50. Computer control of the operations performed within array 1-30 permits automatic, overlapping repetition of these operations within simultaneously used membrane units 17-50. As a result, newly hybridized membranes 17-51 are produced more frequently for detection, and the overall speed of sequencing is increased.

The hybridization of labelled oligomers to the sequencing patterns bound to membrane 17-51 is achieved by flowing a series of fluids over membrane 17-51 that enable hybridization, washing and stripping of membrane 17-51. Such use of solutions is known in the art, as described, for example, in United States Patent 4,942,124.

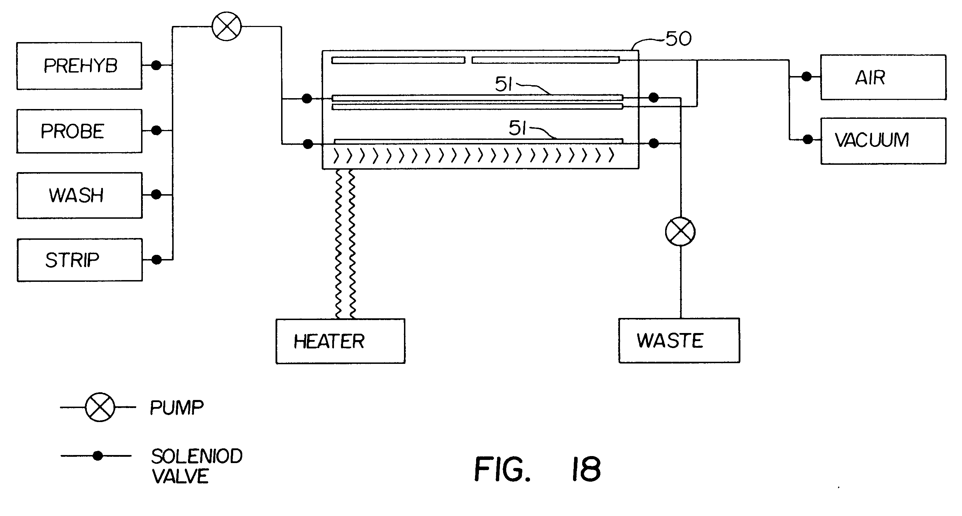

Transportation system 1-21 is provided to transport fluids to and from membrane 17-51 under the force of fluid or air pressure through a series of tubing and valves connected to inlet and outlet tubes 17-54. System 1-21 is separately connected to each membrane unit 17-50 within stations 15-32. Each membrane unit may have a set of computer actuated valves, one for each tube 16-54, for controlling the flow of fluid contained in system 1-21. Similarly, a separate tube and associated valve may be provided for each fluid contained in system 1-21. Alternatively, several membrane units may be linked on a common input line to all fluids, with each membrane unit having a single-valve connection to the end of the input line and each fluid source having a single valve connection to the start of the input line. FIG. 18 generally shows system 1-21 with a common linkage, and, for simplicity, shows the connection of system 1-21 to only two membrane units 18-50 and a membrane 18-51.

System 1-21 also has an associated waste fluid disposal system. Outlet tube 16-54 of each membrane unit is connected to one or more waste reservoirs by means of a waste valve and associated tubing. System 1-21 may be configured so that a single tube 16-54 is used for both inlet and outlet purposes, depending upon the combination of opened inlet and outlet valves of system 1-21.

System 1-21 is also connected to synthesizer 1-20, enabling any synthesized oligomer to be transported to any membrane 16-51 in accordance with the selection determined by computer 1-10. The transportation of the oligomer to a selected membrane 16-51 is ultimately controlled by computer 1-10. However, one or more additional computer devices associated with synthesizer 1-20 or membrane unit array 1-30 may also be employed to control operation of system 1-21. The computer device controls temperature, liquid, air and vacuum flow through the valve system associated with system 1-21.

After hybridization and washing within a membrane unit 16-50 is complete, that membrane unit 16-50 is presented to detector 15-40 to detect the patterns that result from the hybridization of labelled oligomers to the sequencing patterns bound to membrane 16-51.

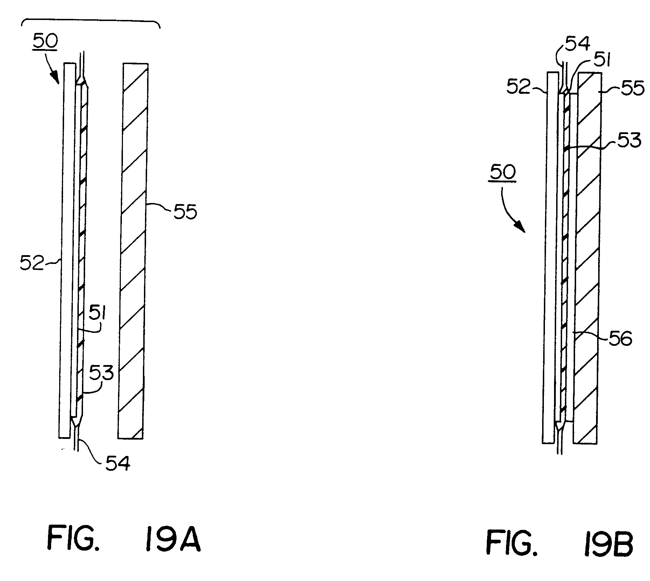

In one embodiment of detector 1-40, detector element 19-56 is used to record the patterns produced by hybridization of radioactively labelled oligomers. The operation of membrane unit 15-50 (16-50) in conjunction with that embodiment of detector 1-40 is shown in FIGS. 19a and 19b. As seen in FIGS. 19a and 19b, cover plate 19-55 is retracted to permit insertion of detector element 19-56 between membrane unit 19-50 (16-50) and plate 19-55 (16-55). Detector element 19-56 in this embodiment may be film, or a Beta-ray detection sheet, for example.

Element 19-56 is first placed over cover sheet 19-53. Plate 19-55 is then moved to the compressed position to place detector element 19-56 in contact with membrane unit 19-50, as shown in FIG. 19b. Contact is maintained until an image of the patterns is produced on element 19-56. Plate 19-55 is then retracted from membrane unit 19-50, and detector element 19-56 is removed. Movement of element 19-56 may be accomplished by robotics, or alternatively, detector element 19-56 may be handled manually. If detector element, 19-56 is film, it may be moved to an automatic developing machine such as an X-Omat before being exposed to detector 1-40.

In the preferred embodiment of detector 1-40, each membrane unit 15-50 can be automatically extended out of membrane unit array 1-30 by a stepper motor or other linear driver to be provided to detector 15-40. Detector 15-40, in turn, can move automatically between stations 15-32 along array 1-30 on rails 15-150, or other such tracks, between exposures. To further enhance the speed of detection, a plurality of detectors 15-40 may be used, enabling patterns on different membranes 16-51 to be observed simultaneously.

Detector 1-40 and detector element 19-56 will, of course, be dependent on the method of labeling used by the oligomer synthesizer. For instance, if the method of labeling employs radioactivity, detector element 19-56 may be a Beta-ray sheet, and detector 1-40 may be a scanning laser beam. If detector element 19-56 is film, detector 1-40 may be a CCD-based scanner.

Alternatively, a chemiluminescent labelled oligomer may be used for hybridization to sequencing patterns bound to membrane 16-51. After the appropriate chemical reactions (see for example, S. Beck et al., supra) light emitting patterns may be read directly from membrane unit 16-50 into a CCD camera for detection, as shown in FIGS. 14 and 15. Regardless of the method of detection, a plurality of detectors 1-40 are preferably included in sequencer 1-1 to detect patterns from different membranes 16-51 simultaneously. Hybridized sequencing patterns detected by detector 1-40 are then transferred to computer 1-10, for instance by a series of electrical signals.

As with the other elements of sequencer 1-1, the computer control of operations within detector 1-40 is ultimately controlled by computer 1-10, but an additional computer, microprocessor or microcontroller that is associated with detector 1-40 may control particular operations within detector 1-40. Communication between detector 1-40 and computer 1-10, or between a separate computer device associated with detector 1-40 and computer 1-10, occurs over line 1-14. Such communication from computer 1-10 informs detector 1-40 of the membrane unit to be detected in conjunction with informing array 1-30 of the membrane unit to make available for detection. Computer 1-10 is informed of the status of the detector, and is ultimately provided with electrical signals generated by detector 1-40 that represent the patterns of hybridized oligomers.

In the preferred embodiment, sequences are constructed in accordance with a walking algorithm by computer 1-10. Computer 1-10 may be provided with a two dimensional pixel image of the sequencing patterns on membrane 16-51 detected by detector 1-40. Such images are then translated into a pattern of letters corresponding to the sequence represented by those images, thus forming sets of short DNA sequences. For example, computer 1-10 may use currently available computer software to translate the graphical images observed by detector 1-40 into a series of corresponding letters A, C, G and T, such as: "Replica" (G. Church, Harvard Medical School, Boston, MA.); BioImage (Ann Arbor, MI); or Protein and DNA Imagewear (Huntington Station, NY.). The sequence from the sequencing lanes of each sample on membrane 16-51 is then combined with sequence obtained from other DNA samples on membrane 16-51 to construct a longer nucleic acid sequence.

Computer 1-10 assembles the sets of nucleic acid sequences based on regions of overlapping bases. A region of overlap may contain for instance, 10 nucleotides, although it is more likely that overlaps of several hundred nucleotides will be generated. The sequence from each membrane 16-51 is then assembled onto previously determined sequence stored in the memory of computer 1-10, again based on regions of overlapping bases. Commercially available software developed for shotgun sequence assembly may be used to combine such short nucleic acid sequences into a final overlapped sequence. Examples of such packages are: GCG (University of Wisconsin); Eugenes (Baylor School of Medicine); and DNA Star (Madison, WI.). Furthermore, the standard software assembly problems are avoided when utilizing the method and apparatus of the present invention, because instead of requiring shotgun matching of random sequences, all that is required is the addition of new sequence in an orderly fashion to the end of previously defined sequence, a much simpler procedure. Because computer 1-10 stores the sequence of the oligomers that were used to hybridize each membrane, assembly may be conducted in a straightforward fashion. Patterns from other membranes hybridized with other oligomers are used to assemble sequence for the opposite strand. If the assembled sequence does not overlap with previously determined sequence, it is stored separately.

For each next iteration of hybridization, computer 1-10 predicts the next set of oligomers based on analysis of the most distant accurate unique sequences within the newly generated sequence. For instance, computer 1-10 may direct synthesis of oligomers complementary to the sequence lying within 20-40 bases from the end of the newly generated sequence. Computer 1-10 directs synthesizer 1-20 to synthesize that next predicted set of oligomers, and selects a corresponding membrane 16-51 from array 1-30 for hybridization with each predicted oligomer. Corresponding membrane 16-51 is selected based on the nucleic acid sequencing patterns bound thereto and on the availability of membrane 16-51 at the time synthesis of the predicted oligomer is complete.

Control of sequencer 1-1 by computer 1-10 enhances the overall speed of sequencing by enabling synthesizer 1-20 to produce sets of multiple oligomers continuously. Computer 1-10 can generally predict a next set of oligomers to be hybridized within some of membrane units 16-50 before completion of synthesis of the previously selected set of oligomers, which are to be hybridized within other of membrane units 16-50. Computer 1-10 accordingly directs synthesizer 1-20 to synthesize that next set of predicted oligomers as soon as synthesizer 1-20 completes synthesis of the previously predicted set of oligomers. Thus, subsequent next sets of oligomers are continually synthesized. As a consequence of this interaction between computer 1-10 and synthesizer 1-20, next predicted oligomers are more likely to be available for hybridization within membrane units 16-50, thereby reducing total sequencing time.

To start the sequencing walks, a variety of starting sequences must be available in order to synthesize a set of oligomers that will be complementary to regions of the unknown sequence. Such oligomers are referred to herein as "starting oligomers." When sequencing unknown genomic DNA directly, known sequences already contained within worldwide data banks may be utilized for this purpose. Additionally or alternatively, the DNA may be cleaved with one or more enzymes recognizing only a small number of nucleotides and shotgun cloned. Short stretches of sequence from multiple clones may then be read using primers homologous to the vector sequences. Similarly, the sequence of starting oligomers for cloned DNA may be determined by sequencing clones containing progressive deletions using primers homologous to vector sequences, such that a short stretch of sequence is read every 1-2 kb.

In the preferred embodiment, starting oligomers of random sequence are synthesized. The length of such random starting oligomers is calculated based on the complexity of the DNA to be sequenced, such that each starting oligomer should hybridize to a DNA sequence occurring only once in the DNA. For example, statistically, any given 11-mer should hybridize only once to DNA having a complexity of two megabases. The random oligomers are then used to probe Southern blots to determine if they identify unique sequences in the DNA. The computer program preferably begins with a collection of approximately 500-1000 random oligomers which identify unique sequence for every megabase of DNA.

Before instructing synthesizer 1-20 to synthesize a predicted oligomer, computer 1-10 will perform various tests on the sequence of that oligomer as a check. Among these tests will be a determination of the secondary structure formed by the sequence. Computer 1-10 will not direct the synthesis of any oligomer capable of forming a hairpin because oligomers that form hairpins may be inefficiently synthesized and may hybridize inefficiently as well. Instead, computer 1-10 will read along the DNA sequence enough bases in the 5' or 3' direction to pick a new short stretch of sequence that will not include the hairpin region and direct synthesis of a new oligomer based on that sequence.

Computer 1-10 will also determine if the next predicted oligomer has previously been used, or if the next predicted oligomer is located within a region in which sequence has already been determined for several hundred bases in both directions. If either of these conditions is found, computer 1-10 does not direct synthesis of that oligomer, but instead directs synthesis of a new starting oligomer.

Computer 1-10 also determines if the newly generated sequence closes a region of DNA. A region of DNA may be closed either by walking inward from both directions, or by having one walk meet the beginning of an adjacent walk in the same direction. When the newly generated sequence is found to close a region of DNA, computer 1-10 shifts its analysis to the most 5' and 3' ends of the region, and directs synthesis of oligomers according to that sequence. Computer 1-10 may also begin an independent determination of sequence in another region, using new starting oligomers.

When the sequence of a given sample is completed, no new oligomers can be made that have not been previously made, or that do not lie within a region of known sequence. In other words, all walks will have come to an end by overlapping other walks, and all starting oligomers will have been used. At this point, the computer will signal that analysis is complete.

Computer 1-10 continuously performs various tests on the newly constructed sequence as it performs the automated multiplex walking. After constructing the sequence derived from each membrane 16-51, the computer will first compare the two complementary DNA strands to search for a mismatch. The most likely cause of mismatches is compression of the pattern of DNA molecules corresponding to sequences containing stretches of C's or G's. This usually occurs because the DNA molecules fold back on themselves so that mobility during size separation does not correlate smoothly with length. Although compressions may occur on both strands, they usually do not occur opposite one another.

When a mismatch between the two strands is found, computer 1-10 may direct synthesis of new oligomers for each strand of DNA close to the trouble spot. These oligomers are then used to probe additional sets of membranes that contain different restriction enzyme digestion products of the same DNA.

Alternatively, computer 1-10 may direct synthesis of modified oligomers capable of hybridizing to sequence ladders generated from DNA that was previously treated with bisulfite. Such treatment causes cytosines in the DNA to be converted to uricils, thereby eliminating compressions. [See, e.g., N. Okada et al., J. Biochem., 91, pp. 1281-91 (1982).] If computer 1-10 cannot resolve a particular mismatch by either of these methods, it will flag the sequence output for later human inspection and intervention. Sequencer 1-1 may continue to operate, however, because other portions of the nucleic acid are still capable of being sequenced.

Certain sequence elements, such as middle and highly repetitive DNA, may not be amenable to sequence analysis by automated sequencer 1-1. Computer 1-10 is able to identify a repeated sequence as a DNA element that has two or more regions apparently flanking it on the 5' and/or 3' sides. Computer 1-10 may not be able to sort the connection across the repeat, and in those cases it will flag the sequence output for later human inspection and intervention.

The higher the complexity of the DNA being sequenced, the more likely it is that such elements will be present. For this reason, it may be advantageous to clone large fragments of high complexity DNA into yeast artificial chromosomes or other vectors, so that repetitive sequences will occur with a decreased frequency. In addition, oligomers derived from multi-copy genes, or derived from conserved portions of genes contained within gene families, may hybridize to multiple sequencing ladders simultaneously, and no discrete sequence will be discernable.

When any of the above situations arise, computer 1-10 will flag the sequence output for later human inspection and intervention so that these regions may be sequenced by other means to determine the connectivity of the sequence across the region. For instance, oligomers derived from the closest unique sequence may be used to screen libraries containing cloned DNA. Once a clone is obtained, it may be

18

mapped and sequenced by methods well known to those skilled in the art, for instance, by Southern blotting and sequencing of progressive deletion mutants. Alternatively, PCR could be used to generate sequence across the repeat. Meanwhile, computer 1-10 would direct the synthesis of a new starting oligomer in order to begin sequence analysis in a different region.

It is also conceivable, particularly when sequencing highly complex genomic DNA directly, that there may exist regions of DNA in which there are no restriction sites. Such regions will not be amenable to sequencing more than several hundred bases from the closest restriction site on each side. In such a case, the oligomer hybridization will not yield discernable sequencing patterns; this is diagnostic of the fact that these oligomers are derived from regions in which there are no restriction sites. Again, upon encountering such a region, computer 1-10 will flag the sequence output for later human inspection and intervention so that this area may be sequenced by other means, for instance, by isolating the DNA with the aid of the diagnostic oligomer, fragmenting it by sonication or shearing, and then sequencing, either before or after cloning.

Thus, it will be apparent that the combination of the four principal components of sequencer 1-1 into a unified apparatus creates a fully automated sequencing machine that rapidly determines the sequence of a nucleic acid molecule by synthesizing multiple oligomers simultaneously, hybridizing oligomers to multiple membranes 51 simultaneously, analyzing the sequence and predicting next sets of oligomers.

It will be apparent to those skilled in the art that the invention described herein can be practiced by other than the embodiments disclosed herein, which are presented for the purposes of illustration and not limitation, and the present invention is limited only by the claims that follow.

Our Ref.: C 891 EP
Case No.: WG-1 EPO
WALTER GILBERT, U.S.A.

```
Appendix A
```

```
.****************************************************************
       Program SOLARSYNTH
  ,
  ,        Solenoid Array Synthesizer.
  ,
  ,        Print*,' Copyright (C) 1990'
  ,        Print*,' President and Fellows of Harvard College'
  ,        Print*,' Steve Kieffer-Higgins and George M. Church'
  ,        Print*,' All rights reserved'
  ,
  ,        Currently set up for standard CE-phosphoramidite DNA synthesis
  ,        and for Omega 992/915 RS232 interface from Omega Engineering.
  ,        To set up vax: set term/perm/eight/speed=9600 txa4
  ,        To set up Omega: connect 50-wire cable from multiplexer to relay
  ,        block.  Connect 5 VDC and 12 VDC supplies to relay block.  Connect
  ,        the 25-pin control cables to the appropriate solenoid and valve arrays
  ,
  ,        Test with TRS-100 TELCOM stat=87I1E : 9600 baud, 7 bits, ignore
  ,        parity, 1 stop bit, line enable
  ,
  ,        Modification History:
  ,
  ,        Adapted from Valve.for 2-mar-87 to SOL4S on 9-Sep-89
  ,        by George Church
  ,
  ,        to SIAS_CPHOS_1 and OMEGA_CPHOS_1 on 25-jun-90
  ,        by Steve Kieffer-Higgins
  ,
  ,        to SOLARSYNTH on 28-Jan-91  by Steve Kieffer-Higgins
  ,
  ,
  ,        Current  setup  piston-col#1 on far right
  ,
  ,        Plate-col . . . 12 11 10 9 8 7 6 5 4 3 2 1
  ,        Plate-col . . . Ox  - Cp - T - - - A G C T
  ,
  ,                        Piston
  ,                        col
  ,                        2  1
  ,
  ,        Piston  8       16 8 --|
  ,        row     7       15 7   |
  ,                6       14 6   |
  ,                5       13 5   |__ solenoid control numbers
  ,                4       12 4   |
  ,                3       11 3   |
  ,                2       10 2   |
  ,                1       9  1 --
  ,
  ,        Lower solenoid tier: 1,3,5,7,10,12,14,16
  ,        Upper solenoid tier: 2,4,6,8,9,11,13,15
  ,
  ,        For this test program only the lower tier is in use:1,3,5,10,12,14
  ,
  ,        Valve block: Upper row, with switches to the left, are #'s 1-12,
  ,        and the lower row is numbered 13-24.  In this run valves 7 & 8
  ,        are not used.  The monomer and tetrazole valves are currently
  ,        sharing relays.
  ,
  ,        Omega relay: This run uses only one relay block consisting of 32
  ,        switches, which is currently the limiting factor for capping as
  ,        well as the total possible oligos which can be made in a single
  ,        run.  These are the current requirements for relays assuming a
  ,        two-part capping reaction:
  ,
  ,        MONOMERS: 12 RELAYS and 16 VALVES
```

Monomers require 1 relay to simultaneously switch of 2 valves, monomer and tetrazole. Each also requires a dedicated rinse and vacuum valves. Thus, each monomer requires 4 valves controlled by 3 relays for a total of 16 valves and 12 relays.

OXIDATION: 3 RELAYS and 3 VALVES
The oxidizer is a one part reagent which requires dedicated rinse and vacuum valves.

CAPPING: 3 RELAYS and 4 VALVES
The capping reagent is a two-part mixture which requires dedicated rinse and vacuum valves.

TCA: 3 RELAYS and 3 VALVES
TCA is a one part reagent which requires dedicated rinse and vacuum valves.

MOTOR: 4 RELAYS
The motor requires 2 relays at 5VDC. Since the relay blocks can switch different voltages in groups of 4 and the valves all require 12VDC, the motor effectively takes 4 relays out of the pool.

TOTAL: 29 RELAYS and 26 VALVES for reagent delivery and trough moveme:

SOLENOIDS: 2 RELAYS EACH
The solenoids require 12VDC to pull up a tip and switching to 5VDC to maintain a hold on the tip. The solenoids reach a temperature greater than 100C when in an array with continuous application of 12VDC, but reach about 35C at 5VDC. Thus each Omega OM915 relay module can control 16 individual solenoids, or a total of 6 OM915 units for 96 solenoids.

The present set up with a single OM915 allows the control of 6 solenoids if we work without a capping reaction. Not capping saves 3 relays and 4 valves. Based on the architecture of the relay blocks, which allows voltages in groups of 4 to be switched, we would be reduced to only 3 different oligos per run were we to incorporate a capping reaction.

RELAY/VALVE ASSIGNMENTS:

| OMEGA RELAY | REAGENT | VALVE |
|---|---|---|
| 1 | tBHP | 19 |
| 2 | TCA | 20 |
| 3 | A monomer | 21 |
| | TET-A | 9 |
| 4 | C monomer | 22 |
| | TET-C | 10 |
| 5 | G monomer | 23 |
| | TET-G | 11 |
| 6 | T monomer | 24 |
| | TET-T | 12 |
| 7 | vac-tBHP | 1 |
| 8 | vac-TCA | 2 |
| 9 | vac-A | 3 |
| 10 | vac-C | 4 |
| 11 | vac-G | 5 |
| 12 | vac-T | 6 |
| 13 | CH3CN-tBHP | 13 |
| 14 | CH3CN-TCA | 14 |
| 15 | CH3CN-A | 15 |
| 16 | CH3CN-C | 16 |
| 17 | CH3CN-G | 17 |
| 18 | CH3CN-T | 18 |
| 25 | Motor – Drive | 25 |
| 26 | Motor – CW/CCW | 26 |

```
19-24   12V     solenoids
27-32   5V      solenoids
```

Each step entails a CH3CN rinse that rinses both the reagent troughs and the tips used in that trough during that step. In this way no additional valves/relays are required to effect a rinse. Additionally, the same control loop can be used for both the chemical and rinse steps with only an update in the control variables. This feature will be most useful in preventing cross-contamination of adjacent tips in the first rinses after monomer addition.

Manual synthesis cycle as performed SKH 14-jun-90

```
        Pos
        1. CH3CN        20"
        2. TCA          5 x 10"
           CH3CN        20"
        3. Base         180"
           CH3CN        20"
        4. Cap          120"
           CH3CN        20"
        5. Oxid         60"
           CH3CN        20"
```

```
integer*4       lenmax ! maximum length of oligos to be made

integer*4       colmax ! maximum number of columns - hardware dependant

integer*4       rowmax ! maximum number of row - hardware dependant

integer*4       stepmax ! number of synthetic steps + 1 (initiation)

integer*4       troughmax ! number of reagent troughs, currently 6
                BUT must be incremented to 7 when capping is added.

integer*4       trough_unit ! the number of motor steps to move 9mm

integer*4       rxnstep ! the number of substeps per synthetic step.
                Currently 3: the actual reaction, vacuuming out the
                trough, and adding rinse.

integer*4       vlvmax! The number of commands that must be sent to
                a given trough, currently 6: open/close reagent,
                vacuum and rinse.

parameter( lenmax=38, colmax=2, rowmax=3, stepmax=5)
parameter( troughmax=6, trough_unit=33, rxnstep=3, vlvmax=6)

real*4          pos(stepmax) !The absolute position of a reagent
                trough within the trough plate - defined by user
                in SOLARSYNTH.TMR

real*4          pumptm(stepmax,rxnstep)!The length of time to hold
                a valve open - defined by user in SOLARSYNTH.TMR

real*4          wait(stepmax)!Reaction time per step - defined by
                user in SOLARSYNTH.TMR

real*4          wait2(stepmax)!Rinse time per step - defined by
                user in SOLARSYNTH.TMR

logical         long !toggles between full length reaction steps.
                When TRUE the times defined in SOLARSYNTH.TMR are
                converted from minutes to seconds; otherwise the
                minutes are treated as seconds.
```

```
      logical           yescheck ! required by GETSTR

      integer*4         ldrvcmmnd, lcmmnd, lreset, lcpureset ! Length of
*                       command strings sent to WRIBOT

      integer*4 —       step ! Control variable which keeps track of where
*                       in the synthesis cycle the program is.

      integer*4         loopbeg, loopend ! the bounds in which STEP operates

      integer*4         mono ! the number of monomers, currently 4

      integer*4         NC, NR ! the actual number of columns and rows of
*                       oligos to be made which will vary with each run

      integer*4         len ! the length of the longest oligo to be made
*                       per run

      integer*4         cyc ! control variable which operates within the
*                       bounds of LEN, above

      integer*4         ld(colmax,rowmax), lu(colmax,rowmax)! length of
*                       commands that raise (lu) and lower (ld) the
*                       solenoids, required by WRIBOT

      integer*4         seqm(colmax,rowmax,lenmax)!integer representation
*                       of the oligos to be made, where A=1, C=2 etc

      character*1       mlis(20) ! reference against which an element of
*                       SEQM above is compared when the program is testing
*                       to see if a solenoid is to drop its tip.  When the
*                       number in the SEQM array matches the subscript of
*                       MLIS, the tip is dropped.

      character*30      Chem(stepmax), chem2(stepmax) !Description of
*                       each step: Chem=rxn, chem2=rinse

      character*38      seq(colmax,rowmax)

      character*60      drive(3) !Holds the commands that drive the motor:
*                       1= pulse, 2=CW, 3=CCW

      character*60      check !dummy var required for GETSTR

      character*60      rxnvlv(troughmax, vlvmax)!holds the open and close
*                       commands for the valves

      character*60      cpureset,reset !Reset commands for Omega CPU and
*                       OM915

      character*60      down(colmax,rowmax), up(colmax,rowmax)! Lift and
*                       drop commands for the solenoids


*********************************************************************************
*     MAINLINE
      call INITIALIZE( chem, chem2, colmax,
     2       cpureset, down, drive, lcpureset, ld, ldrvcmmnd,
     3       len, lenmax, long, loopbeg, loopend, lreset, lcmmnd,
     4       lu, mlis, mono, pumptm, rxnstep,
     5       NC, NR, pos, reset, rowmax, rxnvlv, seq, seqm,
     6       stepmax, trough_unit, troughmax, up, vlvmax, wait, wait2)
```

```
        call getstr(check,'Hit RETURN to begin synthesis.','<CR>',yescheck)

        Do cyc=2,Len
           Do step = loopbeg, loopend

              if (step.eq.2) then
                 call ADD_MONOMER (chem, chem2,
     2           colmax,cyc,down,drive,ld,lenmax,ldrvcmmnd, lcmmnd,
     3           lu, alis, mono, NC, NR, pos, pumptm, rxnstep,
     4           rowmax, rxnvlv, seqm, step, stepmax,
     5           trough_unit, troughmax, up, vlvmax, wait, wait2)

              else
                 call NON_ADDITIVE_CHEMISTRY(chem, chem2,
     2           colmax, down, drive, long, ld,
     3           ldrvcmmnd, lcmmnd, lu, rxnstep,
     4           NC, pos, pumptm, rowmax, rxnvlv, step,
     5           stepmax,troughmax,trough_unit, up,vlvmax, wait, wait2)

              end if
           End do ! step

        End do ! cyc

        call wribot( reset(1:lreset) )

        END !solarsynth

 !********************************************************************************
        subroutine NON_ADDITIVE_CHEMISTRY(chem, chem2,
     2           colmax, down, drive, long, ld,
     3           ldrvcmmnd, lcmmnd, lu, rxnstep,
     4           NC, pos, pumptm, rowmax, rxnvlv, step,
     5           stepmax, troughmax, trough_unit, up, vlvmax, wait, wait2)


 *      coordinates TCA, oxidation and capping steps

        integer*4        colmax,rowmax,stepmax,troughmax,rxnstep,vlvmax

        real             time, delta, oldpos
        real*4           pos(stepmax), pumptm(stepmax, rxnstep)
        real*4           wait(stepmax), wait2(stepmax)
        logical          long
        integer*4        NC, vlv, i, step, trough_unit
        integer*4        ldrvcmmnd, lcmmnd
        integer*4        ld(colmax,rowmax), lu(colmax,rowmax)
        character*30     Chem(stepmax), chem2(stepmax)
        character*60     drive(3)
        character*60     down(colmax,rowmax), up(colmax,rowmax)
        character*60     rxnvlv(troughmax, vlvmax)


        if (step.eq.3) return !cap
        if (step.eq.4) then
               vlv=1  !oxidize
               oldpos=5
        end if
        if (step.eq.5) then
               vlv=2  !TCA
               oldpos=12
        end if

        call MOVEx (oldpos,pos(step), drive, ldrvcmmnd,
     2                           lcmmnd, trough_unit)
```

```
*       fill trough: reagent valve - 1
        call pump(rxnvlv(vlv,1), rxnvlv(vlv,2),lcmmnd,pumptm(step,1))

*       react tips:

        print*,chem(step)
        write(66,'(a)')chem(step)
        delta=0
        time=secnds(0.0)
        print*,'REACTION TIME-',(wait(step)/60),'minutes'
        write(66,'(a,f,a)')'REACTION TIME-',(wait(step)/60),'minutes'
        do while(delta.lt.wait(step))
            delta=secnds(time)
            call dip_all_cols(colmax, down, drive, ld, ldrvcmmnd,lcmmnd,
     2       lu, NC,pos, rowmax, step, stepmax, trough_unit, up)
        end do! while

*       rinse trough

        print*,chem2(step)
        write(66,'(a)')chem2(step)
        delta=0
        time=secnds(0.0)
        print*,'RINSE TIME=',(wait2(step)/60),'minutes'
        write(66,'(a,f,a)')'RINSE TIME=',(wait2(step)/60),'minutes'
        call pump(rxnvlv(vlv,3), rxnvlv(vlv,4), lcmmnd, pumptm(step,3))
        do while (delta.lt.wait2(step))

*       remove reagent:

*       rinse:
        print*,chem2(step)
        write(66,'(a)')chem2(step)
        call pump(rxnvlv(vlv,5), rxnvlv(vlv,6), lcmmnd, pumptm(step,2))

        do i - 1, 5
            call dip_all_cols(colmax, down, drive, ld, ldrvcmmnd,lcmmnd,
     2       lu, NC,pos, rowmax, step, stepmax, trough_unit, up)

        end do !dip
        call pump(rxnvlv(vlv,3), rxnvlv(vlv,4), lcmmnd, pumptm(step,3))
        delta=secnds(time)

        end do !while

        return

        end ! non_additive _chemistry

****************************************************************************
        subroutine INITIALIZE( chem, chem2, colmax,
     2       cpureset, down, drive, lcpureset, ld, ldrvcmmnd,
     3       len, lenmax, long, loopbeg, loopend, lreset, lcmmnd,
     4       lu, mlis, mono, pumptm, rxnstep,
     5       NC, NR, pos, reset, rowmax, rxnvlv, seq, seqm,
     6       stepmax, trough_unit, troughmax, up, vlvmax, wait, wait2)


        integer*4       col,row,colmax,rowmax,stepmax,rxnstep,vlvmax
        real*4          pos(stepmax), pumptm(stepmax, rxnstep)
        real*4          wait(stepmax), wait2(stepmax)
        logical         long, yescheck
        integer*4       troughmax, loopbeg, loopend,stepnum
        integer*4       i, b, bb, q, p, m, mono, trough_unit
```

25

```
integer*4        Len, lenmax, seqm(colmax,rowmax,lenmax)
integer*4        NC, NR, vlv, ldrvcmmnd, lcmmnd, prm
integer*4        ld(colmax,rowmax), lu(colmax,rowmax)
integer*4        lreset, lcpureset, rotn
character*1      mlis(20), newdir, oldir
character*8      waitasec
character*30     Chem(stepmax), chem2(stepmax)
character*38     seq(colmax,rowmax)
character*60     dummyline
character*60     cpureset, reset, halt, CW, CCW
character*60     down(colmax,rowmax), up(colmax,rowmax)
character*60     drive(3), str, check, prmvlv, vacprm
character*60     rxnvlv(troughmax,vlvmax)


*       Colmax & rowmax determine the number of pins used in the program.
*       With only one OM-915 we have room for only 6 individually
*       adressable solenoids, 2 columns x 3 rows. Stepmax = 6, which
*       reflects the initial step of starting the synthesis and 4
*       synthetic reactions: 2=monomer addition, 3=cap ( not used
*       in this version), 4=oxidation, 5=trityl release.  Each step
*       has a CH3CN rinse at the end.


        Loopbeg= 2  ! Start with preloaded detritylated solid phase
        Loopend= 5  ! 4 actual synthesis steps, if we use capping

!       Solenoid columns begin with one on the left.
!       At X=0.0 , pos=1 lines up with col=1, pos=2 with col=2 etc.

        mono = 4 ! number of monomer solutions 4 = ACGT
        call getstr(str,' Long(full) version or rapid check','rapid',long)
        long=.not.long

        mlis(1)='A'
        mlis(2)='C'
        mlis(3)='G'
        mlis(4)='T'

        Chem( 1)= 'M  Start'
        pos( 1)= 8  ! trough is moved to monomer pos from TCA pos

* The following routines establish the timing parameters for each step.
* The user may input any timing parameter as minutes in real
* numbers, ie 3.5 instead of 3:30. Input file follows this format:
*
*       filename: SOLARSYNTH.TMR
*
*       line#    content
*       1.       MINUTES[TAB]TROUGH POS[TAB]TROUGH FILLING TIME
*
*       2.       MONOMER COUPLING
*       3.       rxn time [spaces] trough pos [spaces] time to fill trough
*       4.       MONOMER RINSE
*       5.       rinse time [spaces] time to fill trough
*       6.       MONOMER TROUGH VACUUM
*       7.       time to empty trough,
*
*       8.       CAPPING REACTION
*       9.       rxn time [spaces] trough pos [spaces] time to fill trough
*       10.      CAPPING RINSE
*       11.      rinse time [spaces] time to fill trough
*       12.      CAPPING TROUGH VACUUM
*       13.      time to empty trough
*
```

```
*    14.       OXIDATION
*    15.       rxn time [spaces] trough pos [spaces] time to fill trough
*    16.       OXIDATION RINSE
*    17.       rinse time [spaces] time to fill trough
*    18.       OXIDATION TROUGH VACUUM
*    19.       time to empty trough
*
*    20.       TCA TRITYL RELEASE
*    21.       rxn time [spaces] trough pos [spaces] time to fill trough
*    22.       TCA RINSE
*    23.       rinse time [spaces] time to fill trough
*    24.       TCA TROUGH VACUUM
*    25.       time to empty trough
*
*

          print*,'Opening SOLARSYNTH.TMR for timing parameters...'
          open(11,name='solarsynth.tmr',status='old',readonly)

*         take care of header:
          read(11,'(a)')dummyline
*         read the 4 sets of timing parameters:
          do i= 2, 5
                  read(11,'(a)')chem(i)
          write(66,*)'chem(i)',chem(i)
                  read(11,*)wait(i),pos(i),pumptm(i,1)
          write(66,*)'wait(i),pos(i),pumptm(i,1)',wait(i),pos(i),pumptm(i,1)
                  read(11,'(a)')chem2(i)
                  write(66,*)'chem2(i)',chem2(i)
                  read(11,*)wait2(i),pumptm(i,2)
          write(66,*)'wait2(i),pumptm(i,2)',wait2(i),pumptm(i,2)
                  read(11,'(a)')dummyline
                  read(11,*)pumptm(i,3)
          write(66,*)'pumptm(i,3)',pumptm(i,3)
          end do 11
          if (long) then
              do i=2,5
                  wait(i)=wait(i)*60
                  wait2(i)=wait2(i)*60
              end do
          end if

*         read sequence data:
          print*,'Opening SEQUENCE.IN for synthesis parameters...'
          Open(10,name='sequence.in',status='old',readonly)
          Len=0
          Do col=1,colmax
              Do row = 1, rowmax
                  read(10,'(a)',err=9,end=9)seq(col,row)
!                 determine Len, flip 3' to 5', and translate to monomer #
                  do b=1,lenmax
                      seqm(col,row,b)=0 ! initialize to no monomer coupling
                  end do
                  bb=0
                  do b=lenmax,1,-1
                      do m=1,mono
!                         Check Upper and lowercase ACGT,etc.:
                          If( char(ichar(seq(col,row)(b:b))-32).eq.mlis(m)
     &                        .or.seq(col,row)(b:b).eq.mlis(m) ) then
                              bb=bb+1
                              if(bb.eq.1) write(66,*)' '
                              seqm(col,row,bb)=m
!                             writes a #:1,2,3 or 4,corresponding to a monomer
                              write(66,'(a,4i4,x,a)')' Col,Row,base,m'
     &                            ,col,row,bb,m,seq(col,row)(b:b)
                              goto 1
```

```
        End do

        print*,'CPU RESET'
        write(66,'(a)')'CPU RESET'
        call wribot(cpureset (1:lcpureset))

        print*,'MULTIPLEXER RESET'
        write(66,'(a)')'MULTIPLEXER RESET'
        call wribot(reset (1:lreset))

        NC=col-1
        NR=row-1
        If(NR.le.0) then
            NR=rowmax
            NC=NC-1
        End If

    Initialize all relays to the no power state, then set to "UP".
        Do col = 1,NC
            Do row = 1,rowmax
                If(col.eq.NC.and.row.gt.NR) goto 2
                call wribot( up(col,row)(1:lu(col,row)) )
            End do ! row
        End do ! col

2       continue
        print*,'Enter the number which corresponds to desired operation:'
        print*,' '
        print*,'1. Position trough unit.'
        print*,'2. Prime reagents.'
        print*,'3. Activate solenoids.'
        print*,'4. Initiate synthesis.'
        print*,' '
        call getlint(prm,'Your choice?',999)
        if (prm.eq.999) goto 2
        if (prm.lt.1) goto 2
        if (prm.gt.4) goto 2

        if (prm.eq.1) call position_trough(drive,lcmmnd,ldrvcmmnd,
    2        trough_unit)

        if (prm.eq.2) call prime_reagents(cpureset,lcmmnd,lcpureset,
    2        lreset,reset)

        if (prm.eq.3) call solenoids(colmax,down,ld,lu,NC,rowmax,up)

        if (prm.eq.4) goto 3

        goto 2

3       continue

        return

        end !initialize

****************************************************************************
        subroutine DIP_ALL_COLS (colmax, down, drive, ld, ldrvcmmnd,lcmmnd,
    2        lu, NC, pos, rowmax, step, stepmax, trough_unit, up )

*       Dips all tips one column at a time into a given trough

        integer*4      col, row, rowmax, stepmax, colmax
        real*4         pos(stepmax), newpos
        integer*4      NC, NR, step, trough_unit, ldrvcmmnd, lcmmnd
        integer*4      ld(colmax,rowmax), lu(colmax,rowmax), i,q
```

28

```
        character*8      waitasec
        character*60     down(colmax,rowmax), up(colmax,rowmax), drive(3)

        waitasec='0:00.50'
        Do col = 1,NC
         do i=1, 5
           Do row = 1,rowmax
                 If(col.eq.NC+1.and.row.gt.NR) goto 4
                 Call wribot(down(col,row)(1:ld(col,row)) )
           end do ! row

           call waiter(waitasec)

           do q=1,5
             Do row = 1,rowmax
                 Call wribot(up(col,row)(1:lu(col,row)) )
             end do ! row
           end do !q
           newpos=pos(step)-col
         end do ! i


           if (col.lt.NC) call movex
     2        (pos(step)-col+1,newpos,drive,ldrvcmmnd,lcmmnd,trough_unit)
           if (col.eq.NC) call movex
     2        (pos(step)-col+1,pos(step),drive,ldrvcmmnd,lcmmnd,trough_unit)
     4   End do ! col
        continue
        return

        end ! dip_all_cols

*******************************************************************
        subroutine ADD_MONOMER (chem, chem2,
     2        colmax,cyc,down,drive,ld,lenmax,ldrvcmmnd, lcmmnd,
     3        lu, mlis, mono, NC, NR, pos, pumptm, rxnstep,
     4        rowmax, rxnvlv, seqm, step, stepmax,
     5        trough_unit, troughmax, up, vlvmax, wait, wait2)

*       Coordinates monomer addition

        integer*4        colmax,rowmax,stepmax,lenmax,rxnstep,vlvmax.
        real             delta, time
        real*4           pos(stepmax),pumptm(stepmax,rxnstep)
        real*4           wait(stepmax), wait2(stepmax)
        logical          long
        integer*4        ldrvcmmnd, lcmmnd, step, mono, cyc, q
        integer*4        NC, NR, co, i, troughmax, trough_unit
        integer*4        ld(colmax,rowmax), lu(colmax,rowmax)
        integer*4        seqm(colmax,rowmax,lenmax),firstmono,lastmono
        character*1      mlis(20)
        character*30     chem(stepmax), chem2(stepmax)
        character*60     drive(3)
        character*60     rxnvlv(troughmax, vlvmax)
        character*60     down(colmax,rowmax), up(colmax,rowmax)

*       pump monomers:
        firstmono = 3 !INCREASE TO 4 WITH CAPPING
        lastmono = 6 !INCREASE TO 7 WITH CAPPING

        do i=firstmono, lastmono
           call pump(rxnvlv(i,1), rxnvlv(i,2),lcmmnd,pumptm(step,1))
        end do


*       while there is time: wait
```

```
      call movex(pos(step-1),pos(step),drive,ldrvcmmnd,lcmmnd,trough_unit)
      print*,chem(step)
      write(66,*)chem(step)
      delta=0
      time=secnds(0.0)
      do while (delta.le.wait(step))
          call addition  (cyc, colmax, down, drive, ld,ldrvcmmnd,lcmmnd,
     2       lenmax,lu,mlis,mono, NC, NR, pos, rowmax, seqm, step,
     3       stepmax, troughmax, trough_unit, up)

          delta=secnds(time)
      end do! addititon

*     while there is time : wait2
      print*,chem2(step)
      write(66,*)chem2(step)
      delta=0
      time=secnds(0.0)

*     remove reagent:
      do i= firstmono, lastmono
          call pump(rxnvlv(i,3), rxnvlv(i,4), lcmmnd, pumptm(step,3))
      end do

      do while (delta.le.wait2(step))
*     pump rinse:
          do i= firstmono, lastmono
              call pump(rxnvlv(i,5), rxnvlv(i,6), lcmmnd, pumptm(step,2))
          end do

          do q=1, 5
              call addition (cyc,colmax,down,drive,ld,ldrvcmmnd,lcmmnd,
     2           lenmax,lu,mlis,mono, NC, NR, pos, rowmax, seqm, step,
     3           stepmax, troughmax, trough_unit, up)
          end do ! q
          delta=secnds(time)
          do i= firstmono, lastmono
              call pump(rxnvlv(i,3), rxnvlv(i,4), lcmmnd, pumptm(step,3))
          end do

      end do! rinse

      return

      end ! add monomer

****************************************************************************
      subroutine ADDITION  (cyc,colmax,down, drive,ld,ldrvcmmnd,lcmmnd,
     2       lenmax,lu,mlis,mono,NC, NR, pos, rowmax,seqm,step,stepmax,
     3       troughmax, trough_unit, up)

*     decides which tips drop into which monomer trough

      integer*4      col, row, colmax, rowmax, stepmax, lenmax
      real*4         pos(stepmax), lastep, firstep
      logical        long, drop
      integer*4      b, bb, cyc, dip, loopbeg, loopend, m,mono,step
      integer*4      NC, NR, co, i, c, r, trough_unit, troughmax,q
      integer*4      ld(colmax,rowmax), lu(colmax,rowmax),  lcmmnd
      integer*4      seqm(colmax,rowmax,lenmax),ldrvcmmnd
      character*1    mlis(20)
      character*8    waitasec
      character*60   down(colmax,rowmax), up(colmax,rowmax), drive(3)

      waitasec='00:01.00'
      firstep = pos(step)
```

```
                    End If
                  End do ! m
                End do ! b
              If( Len.lt.bb) Len=bb
              End do_! row
        End do ! col
        col=colmax ! on leave fortran loop index goes +1 beyond limit!
9       continue

*       interface resets:

        write(cpureset,'(a)')'@RESET'//char(13)//char(10)
        write(reset,'(a)')'#1 RESET'//char(13)//char(10)
        lcpureset=8
        lreset=10

*       valve assignments:
*       We are currently set up for 6 reagent troughs, each with dedicated
*       reagent, vacuum & rinse valves.  Capping is not used.  If we add
*       capping later the omega relay block will have to be rewired to
*       reflect this and the control var TROUGHMAX should be increased to 7.
*       The variable q reflects the actual Omega relay number. Once capping
*       has been integrated into the program the wiring harness must be
*       rewired so that relay 1=tBHP, 2=CAP, 3=TCA, 4=A monomer, etc.

        q=1
        do p=1,vlvmax,2
           do i=1, troughmax
              write(rxnvlv(i,p),'(a,i2,a)')'#1,SW',q,'=1'
2             //char(13)//char(10)
              write(rxnvlv(i,p+1),'(a,i2,a)')'#1,SW',q,'=0'
2             //char(13)//char(10)
              q=q+1
           end do ! vlvmax
        end do ! troughmax

*       relays 19 - 24 = solenoid 12V

*       MOTOR - drive req 5V
        write(drive(1),'(a)')'#1,SW25=1;SW25=0'//char(13)//char(10)

*       MOTOR - CW/CCW
        write( drive(2) ,'(a)')'#1,SW26=1'//char(13)//char(10)
        write( drive(3),'(a)')'#1,SW26=0'//char(13)//char(10)

        ldrvcmmnd = 19
        lcmmnd = 11

*       relays 27-32 = solenoid 5V

*       lift & drop commands for the solenoids:

        m=18 !1st 12V solenoid assignment on OM-915 is next, 19
        Do col=1,colmax   ! col = x
           Do row=1,rowmax ! row = y
              ld(col,row)=11 ! 11 characters long
              lu(col,row)=25 ! 25
              m=m+1
              write(down(col,row),'(a,i2,a)')'#1,SW',m+8,'=0'
2 //char(13)//char(10)
*          print*,'COL, ROW, DOWN(col,row)',col ,row, down(col,row)
              write(  up(col,row),'(a,i2,a,i2,a,i2,a)')
2 '#1,SW',m,',',m+8,'=1'//char(13)//char(10)//
3 '#1,SW',m,'=0'//char(13)//char(10)
*          print*,'COL, ROW, UP(col,row)',col ,row, up(col,row)
```

31

```
lastep = pos(step)
drop=.false. !conditional for monomer dip
   NC holds same value as COLMAX:
   Do col = 1, NC+3
         call MOVEx( lastep, firstep-col+1, drive,ldrvcmmnd,
2                    lcmmnd, trough_unit)

         pos(step) for monomers= -3, such that the first
         column of tips stands directly above the 'A' trough.
         When col=2, move is to position -2, etc.

      Do row = 1,rowmax
            bailout conditional:
      If(col.eq.NC+3.and.row.gt.NR) goto 7
            then the last column/row is past the end

            min(mono,col) returns the smallest #, mono, which = 4,
            or col, which increments by 1 from 1 to NC+3
            first pass: m=1; loop is used once:only one column
            could be over the first monomer trough.
            Second pass, m=2; loop used twice, 2 columns are
            over monomer troughs:
      Do m=min(mono,col),1,-1
         co=col-m+1          ! first pass: co=1-1+1 = 1
         if(co.gt.NC) goto 11
         if(co.eq.NC.and.row.gt.NR) goto 11    !bailout conditional

            test to see if the integer held in seqm(co,row,cyc),
            which corresponds to a monomer, is the same as m
            First pass: m=1

         If(seqm(co,row,cyc).eq.m) then
            if it does write to output file & drop that tip:
            write(66,'(a,4i3,x,a)')' Cyc,col,row,co, base',
2                        cyc,col,row,co, mlis(m)
                  call wribot( down(co,row)(1:ld(co,row)) )
            end if !
         end do !m
11       continue
      end do ! row
      call waiter(waitasec)

         lift tips:cmmd to all
      do c=1,NC
         do r=1,rowmax
            if (c.eq.NC.and.r.gt.NR) goto 14
            do q=1,5
             call wribot( up(c,r)(1:lu(c,r)) )
            end do ! q
         end do !row
      end do ! col

14       continue
         lastep=firstep-col+1
         drop=.false.

   End do ! col
7  Continue
   call MOVEx(lastep-1,firstep,drive,ldrvcmmnd,lcmmnd,trough_unit)

   return

   End ! addition
```

```
*********************************************************************
      subroutine wribot(data)
*
*     To output strings of unknown length to serial RS232 port
*     without terminal <CR> or other characters.
*     Version 1.0     Apr 2 1987      GMC
*
      integer*2      ttchan,ttinit,iosb(4)
      integer*4      i,SYS$QIOW,icode,funcw,efn
      integer*4      LIB$GET_EF,LIB$FREE_EF
      character*(*)  data

*     include '($iodef)'
      external IO$_WRITEVBLK,IO$M_NOFORMAT
*
      ttchan=ttinit()
      funcw=ior(%loc(IO$_WRITEVBLK),%loc(IO$M_NOFORMAT))
      icode=LIB$GET_EF(efn)
      if (.not.icode) call LIB$SIGNAL(%VAL(icode))
      icode = SYS$QIOW ( %val(efn),%val(ttchan),%val(
     2 funcw),iosb,,,%ref(data),%val(len(data)),,%val(0),, )
      if (.not.icode) call LIB$SIGNAL(%VAL(icode))
      icode=LIB$FREE_EF(efn)
      if (.not.icode) call LIB$SIGNAL(%VAL(icode))
*
      return
      end


*********************************************************************
      integer*2 function TTINIT()
*
*     returns communication channel number for terminal-like I/O
*     for example through RS232C ports to the arm and stage.
*
      logical        init,yestemp
      integer*2      ttchan
      integer*4      i,SYS$ASSIGN,icode
      character*60   port

      data init /.true./
*     This will keep these and only these values
      save init,ttchan
*     around for use each time this routine is called
*
      if(init) then
          port='txa4:'
!         call getstr(port,' RS232 port 0-3 ',port,yestemp)
          icode = SYS$ASSIGN(port(:5), ttchan,, )
          If (.not.icode) call LIB$SIGNAL(%VAL(icode))
          init=.false.
      end if
      TTINIT = ttchan
      return
      end
*********************************************************************
      subroutine timer_sub()
      include '($syssrvnam)'
      integer*4 status
      status=sys$wake(,)
      return
      end

*********************************************************************
      subroutine waiter(waist)
      character*5 waist
```

```
        include '($syssrvnam)'
        integer*4 interval(2),status

        external timer_sub
        print*,'        ...waiting ',waist
        status=sys$bintim('0 :'//waist, interval)
        print*,'status=',status
        if(.NOT. status) call lib$signal(%val(status))
        status=sys$    setimr( , interval ,timer_sub,)
        if(.NOT. status) call lib$signal(%val(status))
        status=sys$hiber()
        if(.NOT. status) call lib$signal(%val(status))
        return
        end

!***********************************************
        Subroutine MOVEx(oldpos,newpos,drive,ldrvcmmnd,
      2        lcmmnd,trough_unit)

*       Sends a series of pulses to the OM915 to increment stepper
*       motor.  It decides which direction to turn the motor by
*       comparing the old position to new position.

        real            oldpos, newpos
        real            pos, time
        logical         yes,long
        integer*4       lcmmnd, trough_unit, q, i,ldrvcmmnd, delta
        character*8      waitasec
        character*60     str
        character*60     drive(3)



*       drive: 1=pulse on/off, 2=CW, 3=CCW
        delta = int(oldpos-newpos)

*       if delta is positive then rotate CCW, if negative then rotate CW
        if (delta.ge.0) call wribot(drive(3) (1: lcmmnd))
        if (delta.lt.0) then
                call wribot(drive(2) (1: lcmmnd))
                delta=delta*(-1)
        end if!delta.lt.0
        if (delta.eq.0) delta=1

        waitasec='0:00.01'
        do i = delta, 1, -1
          do q=1, trough_unit
                call wribot(drive(1) (1:ldrvcmmnd))
                call waiter(waitasec)
          end do !q
        end do !i


        return

        End     !MOVEx

!***********************************************
        Subroutine Pump(vlvopn, vlvcls, lcmmnd, pumptm)

*       opens and closes a given valve for time value in PUMPTM

        real            delta, time, pumptm
        integer*4       lcmmnd
        character*60     vlvopn, vlvcls
```

34

```
        print*,'pumptm=',pumptm
*       open valves
        call wribot(vlvopn(1:lcmmnd))
*       wait for pumptm:
        delta=0
        time=secnds(0.0)
        do while(delta.lt.pumptm)
            delta=secnds(time)
        end do! while

*       close valves
        call wribot(vlvcls (1:lcmmnd))

        Return

        End !pump

********************************************************************
        subroutine POSITION_TROUGH(drive, lcmmnd, ldrvcmmnd,
     2                  trough_unit)

*       initializes trough to TCA position and allows user to move trough
*       to any of the active trough positions

        real*4          oldpos, newpos
        logical         long, check, yescheck
        integer*4       lcmmnd, ldrvcmmnd, trough_unit, i
        character*8      waitasec
        character*60     drive(3), resp

*       position troughs

96      call getstr(resp,'Initialize the trough? (Y/N)','Y',long)
        if ((resp.eq.'N').or.(resp.eq.'n')) goto 97
        if ((resp.eq.'Y').or.(resp.eq.'y')) goto 95
        goto 96

95      print*,'Position troughs so that column 1 is over the TCA trough:'
        print*,' '
        print*,'1. Turn off power to the stepper motor.'
        print*,'2. Push trough unit ALL THE WAY to the left (motor end).'
        print*,'3. Turn on power to the stepper motor.'
        print*,' '
        print*,'NEVER MANUALLY MOVE THE MOTOR WITH POWER ON!'
        print*,' '
        print*,'Make sure that your fingers are out of the way!'

        call getstr(check,'Press RETURN when done:',yescheck,long)
        call wribot(drive(2) (1:lcmmnd))
        waitasec='0:00.01'

        do i=1,365      !this puts trough 8 (TCA) under col 1
                call wribot(drive(1) (1:ldrvcmmnd))
                call waiter(waitasec)
        end do !i

        oldpos=8

97      print*,' '
        print*,'Enter the number which corresponds to positioning'
        print*,'column 1 over the indicated trough:'
        print*,' '
        print*,'pos     col 2   col 1'
        print*,'0.      T       -'
        print*,'1.      G       T'
```

```
        print*,'2.        C        G'
        print*,'3.        A        C'
        print*,'4.        -        A'
        print*,'8.        -        TCA'
        print*,'9__      TCA       -'
        print*,'10.       -        CAP'
        print*,'11.      CAP       -'
        print*,'12.       -        OX'
        print*,'13.      OX        -'
        print*,'14.      Reinitialize trough '
        print*,'999.     Quit'
        print*,' '
        print*,'Unless 14 is entered the trough will automatically'
        print*,'return to position 8 when you exit this routine.'
        print*,' '

        call get1real(newpos,'Your choice? Current position:',oldpos)

        if (newpos.eq.999) goto 98
        if (newpos.lt.0) goto 97
        if (newpos.eq.14) goto 96
        if (newpos.gt.14) goto 97
        if ((newpos.ge.5).and.(newpos.lt.8)) goto 97
        if (newpos.eq.oldpos) goto 97
        call movex(oldpos,newpos,drive,ldrvcmmnd,lcmmnd,trough_unit)
        oldpos=newpos
        goto 97

98      continue
        if (oldpos.ne.8) then
                newpos=8
                call movex(oldpos,newpos,drive,ldrvcmmnd,lcmmnd,
        2       trough_unit)
        end if

        return

        end !position_trough

************************************************************************
        subroutine PRIME_REAGENTS(cpureset,lcmmnd,lcpureset,lreset,reset)

*       gives user control over reagent valves

        integer*4      prm, lcmmnd, lreset, lcpureset, i
        logical        yescheck
        character*8    waitasec
        character*60   vacprm, prmvlv, cpureset, reset, check

89      print*,' '
        print*,'Vacuum valve will be open with all reagent selections'
        print*,' '
        print*,'Enter the number corresponding to the valve:'
        print*,'1. tBHP            7. tBHP vacuum   13. tBHP rinse'
        print*,'2. TCA             8. TCA vacuum    14. TCA rinse'
        print*,'3. A monomer       9. A vacuum      15. A rinse'
        print*,'4. C monomer      10.C vacuum       16. C rinse'
        print*,'5. G monomer      11.G vacuum       17. G rinse'
        print*,'6. T monomer      12.T vacuum       18. T rinse'
        print*,' '
        call get1int(prm,'Your choice? 999 to quit',999)

        if (prm.eq.999) goto 88 !quit

        if ((prm.gt.18).or.(prm.lt.1)) goto 89 !input out of range
```

```
        if (prm.lt.7) then !reagent valve
            write(vacprm,'(a,i2,a)')'#1,SW',prm+6,'=1'
     2      //char(10)//char(13)
            call wribot(vacprm(1:lcmmnd))
            write(vacprm,'(a,i2,a)')'#1,SW',prm+6,'=0'
     2      //char(10)//char(13)

        end if

        if (prm.gt.12) then !rinse valve
            write(vacprm,'(a,i2,a)')'#1,SW',prm-6,'=1'
     2      //char(10)//char(13)
            call wribot(vacprm(1:lcmmnd))
            write(vacprm,'(a,i2,a)')'#1,SW',prm-6,'=0'
     2      //char(10)//char(13)
        end if

*       if its anything else:
        write(prmvlv,'(a,i2,a)')'#1,SW',prm,'=1'//char(10)//char(13)
        call wribot(prmvlv(1:lcmmnd))
        write(prmvlv,'(a,i2,a)')'#1,SW',prm,'=0'//char(10)//char(13)
        waitasec='0:01.00'
        call getstr(check,'Hit RETURN to close valve','<CR>',yescheck)
        call wribot(prmvlv(1:lcmmnd))
        call waiter(waitasec)
        call wribot(vacprm(1:lcmmnd))
        goto 89

88      continue

        return

        end !prime_reagents

***************************************************************************
        subroutine SOLENOIDS(colmax,down,ld,lu,NC,rowmax,up)

*       gives user direct control over individual solenoids

        integer*4       colmax,rowmax,c,r,NC
        logical         yescheck
        integer*4       lu(colmax,rowmax), ld(colmax,rowmax)
        character*60    up(colmax,rowmax), down(colmax,rowmax)
        character*60    cmmnd

        print*,' '
        print*,'This routine allows you to control individual solenoids.'
        print*,'All tips will automatically lift when you exit the routine.'
        print*,' '

888     print*,'Enter 999 to quit.'
        call getlint(c,'Column:',999)
        if (c.eq.999)goto 885
        if (c.gt.colmax.or.c.lt.1) then
            print*,'Input out of range.'
            goto 888
        end if

887     call getlint(r,'Row:',1)
        if (r.eq.999)goto 885
        if (r.gt.rowmax.or.r.lt.1) then
            print*,'Input out of range.'
            goto 887
        end if

886     call getstr(cmmnd,'U or D:','U',yescheck)
```

37

```
if (cmmnd.eq.'999')goto 885
if ((cmmnd.eq.'U').or.(cmmnd.eq.'u')) then
        call wribot(up(c,r)(1:lu(c,r)))
        goto 888
end if !up
if ((cmmnd.eq.'D').or.(cmmnd.eq.'d')) then
        call wribot(down(c,r)(1:ld(c,r)))
        goto 888
end if !down
print*,'Input out of range.'
goto 886

885     do c=1,NC
            do r=1,rowmax
                call wribot(up(c,r)(1:lu(c,r)))
            end do !r
        end do !c

884     Continue

        return

        end !solenoids

********************************************************************
```

## Claims

1.  An automated nucleic acid sequencer comprising:
    a synthesizer for synthesizing oligomers;
    means for hybridizing synthesized oligomers to sequencing patterns immobilized on membranes;
    means for detecting hybridized sequencing patterns;
    means for analyzing said detected patterns to construct a nucleic acid sequence; and
    means for predicting next oligomers for subsequent synthesis, hybridization, detection and analysis.

2.  The automated sequencer according to claim 1, wherein said synthesizer simultaneously synthesizes at least two oligomers having different sequences.

3.  The automated sequencer according to claim 1 or 2, wherein said means for hybridizing simultaneously hybridizes at least two oligomers having different sequences to respective selected membranes containing immobilized sequencing patterns.

4.  The automated sequencer according to any one of claims 1 to 3, wherein said means for detecting simultaneously detects hybridized sequencing patterns on at least two membranes.

5.  The automated sequencer according to any one of claims 1 to 4, wherein an oligomer is hybridized to sequencing patterns bound to a first membrane at the same time that hybridized sequencing patterns on a second membrane are detected.

6.  The automated sequencer according to any one of claims 1 to 5, wherein a first oligomer is hybridized to sequencing patterns bound to a first membrane at the same time that a second oligomer is synthesized.

7.  An automated nucleic acid sequencer comprising:
    means for predicting oligomers to be synthesized;
    a synthesizer for synthesizing at least two oligomers of predicted sequence simultaneously;
    a plurality of membranes, each membrane containing immobilized sequencing patterns;
    means for selecting at least one of said membranes for hybridization with each of said synthesized oligomers;
    means for hybridizing said synthesized oligomers to the sequencing patterns immobilized on said selected membranes;
    means for detecting hybridized sequencing patterns; and

38

means for analyzing said detected patterns to construct a nucleic acid sequence.

8. The automated sequencer according to claim 7, wherein said means for hybridizing simultaneously hybridizes at least two said oligomers to the sequencing patterns immobilized on respective selected membranes.

9. The automated sequencer according to claim 7 or 8, wherein said means for detecting simultaneously detects hybridized sequencing patterns on at least two membranes.

10. The automated sequencer according to any one of claims 7 to 9, wherein an oligomer is hybridized to the sequencing patterns immobilized on a first membrane at the same time that the hybridized sequencing patterns immobilized on a second membrane are detected.

11. The automated sequencer according to any one of claims 7 to 10, wherein a first oligomer is hybridized to sequencing patterns immobilized on a first membrane at the same time that a second oligomer is synthesized.

12. An automated nucleic acid sequencer comprising:
   means for synthesizing a set of at least two oligomers of predicted sequence simultaneously;
   means for labelling said oligomers;
   an array containing a plurality of membranes, each said membrane containing immobilized sequencing patterns;
   means for transporting each said labelled oligomer to a selected one of said plurality of membranes for hybridization to said immobilized sequencing patterns;
   a detector for detecting hybridized sequencing patterns; and
   a computer
   for determining a portion of the sequence of said nucleic acid from said hybridized sequencing patterns;
   for checking said sequence;
   for predicting a next set of oligomers to be synthesized and hybridized to sequencing patterns to extend the determined sequence;
   for selecting next membranes for hybridization with said predicted next set of oligomers; and
   for directing the operation of said sequencer.

13. The automated sequencer according to claim 12, wherein at least two predicted oligomers are synthesized simultaneously by a single synthesizer.

14. A membrane unit which comprises:
   a backing plate having a substantially planar front face and margins, said membrane being bonded to said face within said margins;
   a flexible cover sheet bonded to said plate at said margins and extending over said face, enclosing said membrane between said face and said sheet; and
   tubes attached to said sheet to form inlet and outlet ports for permitting fluids to flow between said sheet and said membrane.

15. The membrane unit according to claim 14, wherein a cover plate oriented in a plane substantially parallel to the plane of said face opposes said face and is moveable normal to the plane of said face to contact and to move said sheet towards said membrane to limit the separation between said membrane and said sheet when fluid is placed between said membrane and said sheet.

16. The automated sequencer according to claim 12 or 13, comprising a membrane unit according to claim 14 or 15.

17. The automated sequencer according to claim 12 or 13, comprising a membrane unit according to claim 14 or 15, wherein a detection element is inserted between said sheet and said cover plate.

18. The automated sequencer according to claim 12 or 13, wherein said membrane units are adapted to extend from said array to be presented to a detector for detection of hybridized sequencing patterns.

**19.** An automated sequencer for nucleic acid sequencing using a multiplex walking technique comprising:

an oligomer synthesizer for synthesizing and labelling oligomers of at least two sequences simultaneously;

a membrane unit array comprising a plurality of membrane units, each membrane unit comprising a membrane containing immobilized multiplex sequencing patterns, said membrane units being adapted for multiple independent hybridizations with different oligomers;

means for transporting each labelled oligomer from said synthesizer to a selected membrane unit for hybridization;

a detector for detecting hybridized sequencing patterns on at least two membranes simultaneously; and

a computer

for predicting a set of oligomers;

for directing said synthesizer to synthesize and label said oligomers;

for selecting membrane units for hybridization with each said predicted oligomer;

for directing transport of said oligomers to said selected membrane units;

for directing hybridization of said oligomers to multiplex sequencing patterns immobilized on membranes contained within said membrane units;

for analyzing said hybridized multiplex sequencing patterns to determine short nucleic acid sequences, combining said sequences to construct a longer nucleic acid sequence, and checking said longer nucleic acid sequence for error; and

for predicting a next set of oligomers, and selecting next membrane units for hybridization with said next set of oligomers;

wherein one of said oligomers is hybridized in a first selected membrane unit simultaneously with synthesis of one of said next oligomers for hybridization within a second selected membrane unit and presentation of a third hybridized membrane unit to said detector.

**20.** An automated method for nucleic acid sequencing, which comprises the steps of:

synthesizing oligomers;

hybridizing synthesized oligomers to sequencing patterns immobilized on membranes;

detecting hybridized sequencing patterns;

analyzing said detected patterns to construct a nucleic acid sequence; and

predicting the sequence of next oligomers for subsequent synthesis, hybridization, detection and analysis.

**21.** The method according to claim 20, wherein oligomers having different sequences are synthesized simultaneously.

**22.** The method according to claim 20 or 21, wherein at least two oligomers having different sequences are hybridized simultaneously to respective selected membranes containing immobilized sequencing patterns.

**23.** The method according to any one of claims 20 to 22, wherein at least two membranes are simultaneously presented for detection of hybridized sequencing patterns.

**24.** The method according to any one of claims 20 to 23, wherein at least two steps occur simultaneously on respective of said membranes.

**25.** The method according to any one of claims 20 to 24, wherein an oligomer is hybridized to sequencing patterns immobilized on a first membrane at the same time that the hybridized sequencing patterns on a second membrane are detected.

**26.** The method according to any one of claim 20 to 25, wherein a first oligomer is hybridized to sequencing patterns immobilized on a first membrane at the same time that a second oligomer is synthesized.

**27.** An automated method for nucleic acid sequencing using a plurality of membranes, each membrane containing immobilized sequencing patterns, which comprises the steps of:

predicting oligomers to be synthesized;

40

synthesizing at least two oligomers simultaneously;

selecting at least one of said membranes to correspond to each of said synthesized oligomers;

hybridizing said synthesized oligomers to the sequencing patterns immobilized on said selected membranes;

detecting hybridized sequencing patterns;

analyzing said detected patterns to construct a nucleic acid sequence.

28. The method according to claim 27, wherein at least two said synthesized oligomers are simultaneously hybridized to the sequencing patterns immobilized on at least two respective selected membranes.

29. The method according to claim 27 or 28, wherein the hybridized sequencing patterns on at least two membranes are simultaneously detected.

30. The method according to any one of claims 27 to 29, wherein at least two steps occur simultaneously on respective of said plurality of membranes.

31. The method according to any one of claims 27 to 30, wherein an oligomer is hybridized to the sequencing patterns immobilized on a first membrane at the same time that the hybridized sequencing patterns on a second membrane are detected.

32. The method according to any one of claims 27 to 31, wherein a first oligomer is hybridized to sequencing patterns immobilized on a first membrane at the same time that a second oligomer is synthesized.

33. An automated method for nucleic acid sequencing with a multiplex walking technique using a plurality of membrane units, each containing a membrane upon which multiplex sequencing patterns have been immobilized, which comprises the steps of:

predicting a set of oligomers for synthesis and hybridization;

selecting membrane units for hybridization with said predicted oligomers, each said selected membrane unit corresponding to one of said predicted oligomers;

synthesizing oligomers of at least two different sequences simultaneously;

eluting, purifying and labelling said synthesized oligomers;

transporting each labelled oligomer to a selected membrane unit for hybridization;

hybridizing said transported oligomers to sequencing patterns immobilized on membranes contained within respective selected membrane units;

detecting the hybridized sequencing patterns on at least two membranes simultaneously;

analyzing each said detected pattern to determine a short nucleic acid sequence;

combining said short nucleic acid sequences to construct a longer nucleic acid sequence;

checking said longer nucleic acid sequence for error; and

predicting a next set of oligomers and selecting a next set of membrane units for hybridization with said next set of oligomers, wherein one of said predicted oligomers is hybridized in a first specified membrane unit simultaneously with synthesis of one of said next predicted oligomers for hybridization within a second specified membrane unit and with detection of hybridized sequencing patterns in a third membrane unit.

34. The use of nucleic acid sequence information generated by a method according to any one of claims 20-33, to prepare a nucleic acid molecule having at least a portion of its sequence based on said nucleic acid sequence data.

35. The use of a nucleic acid molecule having at least a portion of its sequence based on nucleic acid sequence information generated by a method according to any one of claims 20-33.

FIG. 1

FIG. 2

FIG. 3

EP 0 514 927 A1

EP 0 514 927 A1

FIG. 4B    FIG. 4A    FIG. 4C

FIG. 4D

44

FIG. 5

FIG. 6

FIG. 7

FIG. 8

FIG. 9

FIG. 10

FIG. 11

YELLOW
ORANGE
RED
BLACK
WHITE/YELLOW
WHITE/ORANGE
WHITE/RED
WHITE/BLACK

60

160

20

50

50a

ROW

COLUMN

40

FIG. 12A

FIG. 12B

FIG. 12C

FIG. 13

FIG. 14

FIG. 15

FIG. 16

FIG. 17A

FIG. 17B

PREHYB

PROBE

WASH

STRIP

50

51

51

AIR

VACUUM

HEATER

WASTE

⊗ — PUMP

• — SOLENIOD VALVE

FIG. 18

EP 0 514 927 A1

FIG. 19A

FIG. 19B

European Patent
Office

EUROPEAN SEARCH REPORT

Application Number

EP    92 10 8687

PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 303 459 (PRESIDENT AND FELLOWS OF HARVARD COLLEGE)<br>* column 6, line 59 - column 12, line 44; figure 7 * | 14,15 | C12Q1/68<br>//G01N35/00 |
| Y | | 1 | |
| A | | 2-12,<br>16-18 | |
| D | & US-A-4 942 124<br>--- | | |
| Y | WO-A-9 106 678 (SRI INTERNATIONAL)<br>* page 11, line 28 - page 18, line 23; claim 50; figure 4 * | 1 | |
| A | | 7,12 | |
| D,Y | ---<br>PROCEEDINGS OF THE NATIONAL ACADEMY OF SCIENCES OF USA.<br>vol. 86, September 1989, WASHINGTON US<br>pages 6883 - 6887;<br>O.OHARA ET AL.: 'Direct genomic sequencing of bacterial DNA: The pyruvate kinase I gene of Escherichia coli'<br>* the whole document * | 20 | |
| | | | TECHNICAL FIELDS SEARCHED (Int. Cl.5) |
| A | | 1,7,12,<br>19 | C12Q<br>G01N |
| | --- | | |
| Y | BIOTECHNOLOGY<br>vol. 8, December 1990, NEW YORK US<br>pages 1251 - 1256;<br>W.BAINS: 'Alternative routes through the genome'<br>* page 1252, column 1, paragraph 3 - page 1256, paragraph 2; figures 3,6 * | 20 | |
| A | | 1 | |
| X | ---<br>DE-A-3 635 966 (HOEFER SCIENTIFIC INSTRUMENTS)<br>* the whole document * | 14,15 | |
| A | ---<br>DE-A-3 306 770 (SHIMADZU CORP.)<br>* the whole document *<br>--- | 1 | |
| | -/-- | | |

The present search report has been drawn up for all claims

| Place of search | Date of completion of the search | Examiner |
|---|---|---|
| BERLIN | 04 SEPTEMBER 1992 | DE KOK A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

EUROPEAN SEARCH REPORT

Application Number

EP    92 10 8687
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | DE-A-3 805 808 (EUROPÄISCHES LABORATORIUM FÜR MOLEKULARBIOLOGIE (EMBL)) <br> * the whole document * <br> --- | 1,12 | |
| T | SCIENCE. <br> vol. 254, no. 5028, 4 October 1991, LANCASTER, PA US <br> pages 59 - 67; <br> T.HUNKAPILLER ET AL.: 'Large-scale and automated DNA sequence determination' <br> * the whole document * <br> ----- | 1-31 | |
| | | | TECHNICAL FIELDS SEARCHED (Int. Cl.5 ) |

The present search report has been drawn up for all claims

| Place of search | Date of completion of the search | Examiner |
|---|---|---|
| BERLIN | 04 SEPTEMBER 1992 | DE KOK A.J. |